# EUROPEAN PATENT SPECIFICATION

(11) **EP 0 602 179 B1**
(45) Date of publication and mention of the grant of the patent: **30.06.1999**
(21) Application number: 92920075.6
(22) Date of filing: 02.09.1992
(51) Int. Cl.: C11D 1/65, C11D 1/86, C11D 1/94, C11D 17/00

(54) **DETERGENT COMPOSITIONS CONTAINING CALCIUM AND POLYHYDROXY FATTY ACID AMIDE**
CALCIUM UND POLYHYDROXYFETTSÄUREAMID ENTHALTENDEN WASCHMITTELZUSAMMENSETZUNGEN
COMPOSITIONS DETERGENTES CONTENANT DU CALCIUM ET UN AMIDE D'ACIDE GRAS POLYHYDROXY

(30) Priority: 06.09.1991 US 755900
(43) Date of publication of application: 22.06.1994
(73) Proprietor: THE PROCTER & GAMBLE COMPANY, Cincinnati, Ohio 45202 (US)
(72) Inventor: OFOSU-ASANTE, Kofi, Cincinnati, Ohio 45224-1111 (US); Willman, Kenneth William, Fairfield, Ohio 45014 (US); FOLEY, Peter, Robert 18 Bowsden Terrace, Newcastle-Upon-Tyne NE3 1RX (US)
(74) Representative: Gibson, Tony Nicholas
(86) International application number: US9207401
(87) International publication number: WO9305132

(56) References cited:
- EP-A- 0 181 212
- EP-A- 0 220 676
- EP-A- 0 285 768
- EP-A- 0 399 752
- WO-A-92/06156
- WO-A-92/06158
- WO-A-92/06171
- WO-A-92/08777
- FR-A- 1 580 491
- GB-A- 2 058 823
- US-A- 2 717 894
- US-A- 2 965 576

## Description

### TECHNICAL FIELD

The present invention relates to detergent compositions containing anionic sulfate surfactant, polyhydroxy fatty acid amide, and calcium ions. Preferred are stable liquid detergent compositions containing N-methyl glucamide, alkyl ethoxy sulfate and lime soap dispersing agent, and calcium ions, or calcium and magnesium ions.

### BACKGROUND OF THE INVENTION

It has been found that the addition of calcium to detergent compositions containing polyhydroxy fatty acid amide and anionic sulfate surfactant can improve the removal of greasy soils while delivering good hand mildness. When, for example, calcium ions are added to light duty liquid dishwashing compositions containing polyhydroxy fatty acid amide (a nonionic surfactant) and anionic sulfate surfactant, surprisingly improved greasy soil removal from dishes is found. This improvement is better than that which is achievable with analogous magnesium-containing light duty liquids.

Calcium is difficult to formulate in a stable liquid composition without a chelating agent, but chelating agents sequester divalent ions, including calcium, from the product or the wash solution. Those divalent ions are then unavailable for cleaning. Thus, being able to formulate a stable calcium-containing detergent composition without or with only a small amount of a chelating agent is a definite benefit. The greasy soil removal benefit, though, exists whether or not the composition is stable.

It has been found that the addition of lime soap dispersing agent ("LSDA") at a certain level to a liquid detergent composition (containing anionic sulfate surfactant and polyhydroxy fatty acid amide) can provide a stable composition with good sudsing performance, greasy soil removal, and mildness to the hands.

Ethoxylated alkyl sulfate with more than 2.5 moles of ethylene oxide per molecule can be used to achieve a stable liquid composition. It is believed that this is because alkyl ethoxy sulfate with higher ethylene oxide itself serves as an LSDA. No extra LSDA is required. Unfortunately, ethoxylated alkyl sulfate with high levels of ethylene oxide tend to compromise sudsing. Sudsing is of course desirable in a light duty liquid detergent composition.

It has also been found that product stability can be further improved by the addition of magnesium to the calcium-containing compositions. This is in addition to the sudsing and cleaning benefits attributable to magnesium.

WO 92/08777 discloses light duty liquid or gel dishwashing detergent compositions comprising alkyl ethoxy carboxylate surfactant mixture and calcium and magnesium ions for grease removal and skin mildness.

Calcium is disclosed for use in certain detergent compositions in U.S. Patent 5,030,378, Venegas, issued July 9, 1991.

The use of anionic sulfate surfactants in detergent compositions is known in the art. For example. U.S. Patent 4,435.317, Gerritson et al., issued March 6, 1984, discloses liquid detergent compositions which contain alkyl sulfate, alkyl ether sulfate and alkylbenzene sulfonate surfactants. U.K. Patent Specification 809,060, published February 18, 1959, discloses detergent compositions containing a sulfate or sulfonate surfactant with a particular polyhydroxy fatty acid amide.

The polyhydroxy fatty acid amide component contained in the composition of the present invention is also known in the art, as are several of its uses. N-acyl, N-methyl glucamides, for example, are disclosed by J. W. Goodby, M. A. Marcus, E. Chin, and P. L. Finn in "The Thermotropic Liquid-Crystalline Properties of Some Straight Chain Carbohydrate Amphiphiles," Liquid Crystals, 1988, Volume 3, No. 11, pp. 1569-1581, and by A. Muller-Fahrnow, V. Zabel, M. Steifa, and R. Hilgenfeld in "Molecular and Crystal Structure of a Nonionic Detergent: Nonanoyl-N-methylglucamide," J. Chem. Soc. Chem. Commun., 1986, pp. 1573-1574.

The use of N-alkyl glucamides (glucosamides) in detergent compositions has also been disclosed. For example, U.S. Patent 2,965,576, Wilson, issued December 20, 1960, and U.K. Patent Specification 809,060, referred to above, relate to detergent compositions containing anionic surfactants and certain amide surfactants, which can include N-methyl glucamide as a low temperature suds enhancing agent.

Lime soap dispersing agents and their ability to prevent soap from precipitating in hard water is discussed in N.M. Linfield's Surfactant Science Series, Vol. 7. pp. 1-10, Marcel Dekker Inc., NY, 1976. U.S. Patent 3,767,584, Hirst, patented October 23, 1973, discloses detergent compositions containing higher fatty acid soaps and lime soap dispersing agents to reduce lime scum formation.

None of these publications disclose a detergent composition containing anionic sulfate surfactant, polyhydroxy fatty acid amide, and calcium for improved greasy soil removal and skin mildness. They do not disclose stable liquid detergent compositions containing alkyl ethoxy sulfate, lime soap dispersing agent, calcium ions, and polyhydroxy fatty acid amide, with or without magnesium ions.

### SUMMARY OF THE INVENTION

The present invention concerns detergent compositions which comprise:
(a) from about 3% to about 95% of anionic sulfate surfactant;
(b) from about 3% to about 40% of polyhydroxy fatty acid amide having the formula: 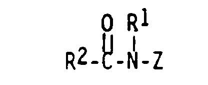 wherein R¹ is hydrogen, C₁-C₄ hydrocarbyl, 2-hydroxyethyl, 2-hydroxypropyl, or mixtures thereof; R² is C₅-C₃₁ hydrocarbyl; and Z is a polyhydroxy-hydrocarbyl having a linear hydrocarbyl chain with at least three hydroxyl groups directly connected to the chain, or an alkoxylated derivative thereof; and
(c) from about 0.1% to about 3% of calcium ions.

Also included are methods for cleaning soiled dishes comprising treating the dishes with these detergent compositions.

### DESCRIPTION OF THE INVENTION

The detergent compositions of the present invention comprise, by weight of the composition: (a) from about 3% to about 95% of anionic sulfate surfactant; (b) from about 3% to about 40% of polyhydroxy fatty acid amide having the formula: 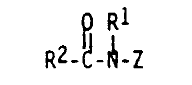 wherein R¹ is hydrogen, C₁-C₄ hydrocarbyl, 2-hydroxyethyl, 2-hydroxypropyl, or mixtures thereof; R² is C₅-C₃₁ hydrocarbyl; and Z is a polyhydroxy-hydrocarbyl having a linear hydrocarbyl chain with at least three hydroxyl groups directly connected to the chain, or an alkoxylated derivative thereof; and (c) from about 0.1% to about 3% of calcium ions. Preferred are stable liquid detergent compositions containing alkyl ethoxy sulfate, lime soap dispersing agent, calcium ions, magnesium ions, and polyhydroxy fatty acid amide.

The three essential ingredients are described below, as are lime soap dispersing agent and magnesium. The detergent compositions of the present invention are preferably in the form of either a liquid or a gel, most preferably a light duty liquid dishwashing detergent composition.

### A. Anionic Sulfate Surfactant

The detergent compositions of the present invention comprise from about 3% to about 95%, more preferably from about 5% to about 60%, most preferably from about 10% to about 40%, by weight of one or more anionic sulfate surfactants. The anionic sulfate surfactant may be any organic sulfate surfactant. It is preferably selected from the group consisting of C₁₀-C₁₆ alkyl sulfate which has been ethoxylated with from about 0.5 to about 20 moles of ethylene oxide per molecule, C₉-C₁₇ acyl-N-(C₁-C₄ alkyl) glucamine sulfate, -N-(C₂-C₄ hydroxyalkyl) glucamine sulfate, and mixtures thereof. More preferably, the anionic sulfate surfactant is a C₁₀-C₁₆ alkyl sulfate which has been ethoxylated with from about 0.5 to about 20, preferably from about 0.5 to about 12, moles of ethylene oxide per molecule.

Alkyl ethoxy sulfate surfactants comprise a primary alkyl ethoxy sulfate derived from the condensation product of a C₁₀-C₁₆ alcohol with an average of from about 0.5 to about 20, preferably from about 0.5 to about 12, ethylene oxide groups. The C₁₀-C₁₆ alcohol itself is commercially available. C₁₂-C₁₄ alkyl sulfate which has been ethoxylated with from about 3 to about 10 moles of ethylene oxide per molecule is preferred. For compositions containing C₁₀-₁₆ alkyl sulfate which has been ethoxylated with from about 0.5 to about 2.5 moles of ethylene oxide per molecule, a lime soap dispersing agent is added for a stable composition.

Conventional base-catalyzed ethoxylation processes to produce an average degree of ethoxylation of 12 result in a distribution of individual ethoxylates ranging from 1 to 15 ethoxy groups per mole of alcohol, so that the desired average can be obtained in a variety of ways. Blends can be made of material having different degrees of ethoxylation and/or different ethoxylate distributions arising from the specific ethoxylation techniques employed and subsequent processing Steps such as distillation.

Anionic sulfate surfactants include the C₉-C₁₇ acyl-N-(C₁-C₄ alkyl) and -N-(C₁-C₂ hydroxyalkyl) glucamine sulfates, preferably those in which the C₉-C₁₇ acyl group is derived from coconut or palm kernel oil Lime soap dispersing agent can be added, especially to the longer chain length glucamine sulfates for improved product stability (e.g., where C₉-C₁₇ acyl is palm kernel oil). These materials can be prepared by the method disclosed in U.S. Patent 2,717,894, Schwartz, issued September 13, 1955.

The counterion for the anionic surfactant component is preferably selected from calcium, sodium, potassium, magnesium, ammonium or alkanol-ammonium, and mixtures thereof, with calcium and magnesium being preferred for cleaning and sudsing, respectively.

The detergent compositions herein preferably comprise from about 5% to about 65%, preferably from about 20% to about 40%, by weight of a surfactant mixture comprising the anionic sulfate surfactant and the polyhydroxy fatty acid amide surfactant.

### B. Polyhydroxy Fatty Acid Amide

The compositions of the present invention also comprise from about 3% to about 40%, preferably from about 5% to about 30%, more preferably from about 8% to about 25%, by weight of one or more polyhydroxy fatty acid amides having the structural formula: 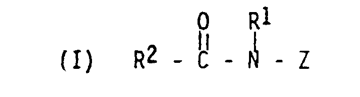 wherein: R¹ is H, C₁-C₄ hydrocarbyl, 2-hydroxy ethyl, 2-hydroxy propyl, or a mixture thereof, preferably C₁-C₄ alkyl, more preferably C₁ or C₂ alkyl, most preferably C₁ alkyl (i.e., methyl); and R² is a C₅-C₃₁ hydrocarbyl, preferably straight-chain C₇-C₁₉ alkyl or alkenyl, more preferably straight-chain C₉-C₁₇ alkyl or alkenyl, most preferably straight-chain C₁₁-C₁₇ alkyl or alkenyl, or mixture thereof; and Z is a polyhydroxyhydrocarbyl having a linear hydrocarbyl chain with at least 3 hydroxyls directly connected to the chain, or an alkoxylated derivative (preferably ethoxylated or propoxylated) thereof. Z preferably will be derived from a reducing sugar in a reductive amination reaction; more preferably Z is a glycityl. Suitable reducing sugars include glucose, fructose, maltose, lactose, galactose, mannose, and xylose. As raw materials, high dextrose corn syrup, high fructose corn syrup, and high maltose corn syrup can be utilized as well as the individual sugars listed above. These corn syrups may yield a mix of sugar components for Z. It should be understood that it is by no means intended to exclude other suitable raw materials. Z preferably will be selected from the group consisting of -CH₂-(CHOH)ₙ-CH₂OH, -CH(CH₂OH)-(CHOH)ₙ₋₁-CH₂OH, -CH₂-(CHOH)₂(CHOR')(CHOH)-CH₂OH, where n is an integer from 3 to 5, inclusive, and R' is H or a cyclic or aliphatic monosaccharide. and alkoxylated derivatives thereof. Most preferred are glycityls wherein n is 4, particularly -CH₂-(CHOH)₄-CH₂OH.

In Formula (I), R¹ can be, for example, N-methyl, N-ethyl, N-propyl, N-isopropyl, N-butyl, N-2-hydroxy ethyl, or N-2-hydroxy propyl.

R²-CO-N< can be, for example, cocamide, stearamide, oleamide, lauramide, myristamide, capricamide, palmitamide, tallowamide, etc.

Z can be 1-deoxyglucityl, 2-deoxyfructityl, 1-deoxymaltityl, 1-deoxylactityl, 1-deoxygalactityl, 1-deoxymannityl, 1-deoxymaltotriotityl, etc.

The most preferred polyhydroxy fatty acid amide has the general formula 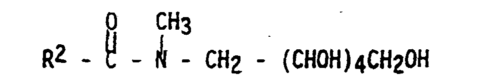 wherein R² is a straight chain C₁₁-C₁₇ alkyl or alkenyl group.

### Method of Preparation

In general, polyhydroxy fatty acid amides can be made by reacting an alkyl amine with a reducing sugar in a reductive amination reaction to form a corresponding N-alkyl polyhydroxyamine, and then reacting the N-alkyl polyhydroxyamine with a fatty aliphatic ester or triglyceride in a condensation/amidation step to form the N-alkyl, N-polyhydroxy fatty acid amide product. Processes for making compositions containing polyhydroxy fatty acid amides are disclosed, for example, in G.B. Patent Specification 809,060, published February 18, 1959, U.S. Patent 2,965,576, issued December 20, 1960 to E. R. Wilson, and U.S. Patent 2,703,798, Anthony M. Schwartz, issued March 8, 1955, and U.S. Patent 1,985,424, issued December 25, 1934 to Piggott.

In one process for producing N-alkyl or N-hydroxyalkyl, N-deoxyglycityl fatty acid amides wherein the glycityl component is derived from glucose and the N-alkyl or N-hydroxy- alkyl functionality is N-methyl, N-ethyl, N-propyl, N-butyl, N-hydroxyethyl, or N-hydroxypropyl, the product is made by reacting N-alkyl- or N-hydroxyalkyl-glucamine with a fatty ester selected from fatty methyl esters, fatty ethyl esters, and fatty triglycerides in the presence of a catalyst selected from the group consisting of alkali metal alkoxide, trilithium phosphate, trisodium phosphate, tripotassium phosphate, tetrasodium pyrophosphate, pentapotassium tripolyphosphate, lithium hydroxide, sodium hydroxide, potassium hydroxide, calcium hydroxide, lithium carbonate, sodium carbonate, potassium carbonate, disodium tartrate, dipotassium tartrate, sodium potassium tartrate, trisodium citrate, tripotassium citrate, sodium basic silicates, potassium basic silicates, sodium basic aluminosilicates, and potassium basic aluminosilicates, and mixtures thereof. The amount of catalyst is preferably from about 0.5 mole % to about 50 mole %, more preferably from about 2.0 mole % to about 10 mole %, on an N-alkyl or N-hydroxyalkyl-glucamine molar basis. The reaction is preferably carried out at from about 138°C to about 170°C for typically from about 20 to about 90 minutes. When triglycerides are utilized in the reaction mixture as the fatty ester source, the reaction is also preferably carried out using from about 1 to about 10 weight % of a phase transfer agent, calculated on a weight percent basis of total reaction mixture, selected from saturated fatty alcohol polyethoxylates, alkylpolyglucosides, linear glucamide surfactant, and mixtures thereof.

Preferably, this process is carried out as follows:
(a) preheating the fatty ester to about 138°C to about 170°C;
(b) adding the N-alkyl or N-hydroxyalkyl glucamine to the heated fatty acid ester and mixing to the extent needed to form a two-phase liquid/liquid mixture;
(c) mixing the catalyst into the reaction mixture; and
(d) stirring for the specified reaction time.

Also preferably, from about 2% to about 20% of preformea linear N-alkyl/N-hydroxyalkyl, N-linear glucosyl fatty acid amide product is added to the reaction mixture, by weight of the reactants, as the phase transfer agent if the fatty ester is a triglyceride. This seeds the reaction, thereby increasing reaction rate.

The polyhydroxy "fatty acid" amide materials used herein also offer the advantages to the detergent formulator that they can be prepared wholly or primarily from natural, renewable, non-petrochemical feedstocks and are degradable. They also exhibit low toxicity to aquatic life.

It should be recognized that along with the polyhydroxy fatty acid amides of Formula (I), the processes used to produce them will also typically produce quantities of nonvolatile by-product The level of these by-products will vary depending upon the particular reactants and process conditions, but are preferably kept to a minimum.

### Alternate Method

An alternate method for preparing the polyhydroxy fatty acid amides used herein is as follows. A reaction mixture consisting of 84.87g. fatty acid methyl ester (source: Procter & Gamble methyl ester CE1270), 75g. N-methyl-D-glucamine (source: Aldrich Chemical Company M4700-0), 1.04g. sodium methoxide (source: Aldrich Chemical Company 16,499-2), and 68.51g. methyl alcohol is used. The reaction vessel comprises a standard reflux set-up fitted with a drying tube, condenser and stir bar. In this procedure, the N-methyl glucamine is combined with methanol with stirring under argon and heating is begun with good mixing (stir bar; reflux). After 15-20 minutes, when the solution has reached the desired temperature, the ester and sodium methoxide catalyst are added. Samples are taken periodically to monitor the course of the reaction, but it is noted that the solution is completely clear by 63.5 minutes. It is judged that the reaction is, in fact, nearly complete at that point. The reaction mixture is maintained at reflux for 4 hours. After removal of the methanol, the recovered crude product weighs 156.16 grams. After vacuum drying and purification, an overall yield of 106.92 grams purified product is recovered. However, percentage yields are not calculated on this basis, inasmuch as regular sampling throughout the course of the reaction makes an overall percentage yield value meaningless. The reaction can be carried out at 80% and 90% reactant concentrations for periods up to 6 hours to yield products with extremely small by-product formation.

The following is not intended to limit the invention herein, but is simply to further illustrate additional aspects of the technology which may be considered by the formulator in the manufacture of a wide variety of detergent compositions using the polyhydroxy fatty acid amides.

It will be readily appreciated that the polyhydroxy fatty acid amides are, by virtue of their amide bond, subject to some instability under highly basic or highly acidic conditions. While some decomposition can be tolerated, it is preferred that these materials not be subjected to pH's above about 11, preferably 10, nor below about 3 for unduly extended periods. Final product pH (liquids) is typically 6.0-9.0.

During the manufacture of the polyhydroxy fatty acid amides it will typically be necessary to at least partially neutralize the base catalyst used to form the amide bond. While any acid can be used for this purpose, the detergent formulator will recognize that it is a simple and convenient matter to use an acid which provides an anion that is otherwise useful and desirable in the finished detergent composition. For example, citric acid can be used for purposes of neutralization and the resulting citrate ion (*ca.* 1%) be allowed to remain with a *ca.* 40% polyhydroxy fatty acid amide slurry and be pumped into the later manufacturing stages of the overall detergent-manufacturing process. The acid forms of materials such as oxydisuccinate, nitrilotriacetate, ethylenediaminetetraacetate, tartrate/succinate, and the like, can be used similarly.

The polyhydroxy fatty acid amides derived from coconut alkyl fatty acids (predominantly C₁₂-C₁₄) are more soluble than their tallow alkyl (predominantly C₁₆-C₁₈) counterparts. Accordingly, the C₁₂-C₁₄ materials are somewhat easier to formulate in liquid compositions, and are more soluble in cool-water laundering baths. However, the C₁₆-C₁₈ materials are also quite useful, especially under circumstances where warm-to-hot wash water is used. Indeed, the C₁₆-C₁₈ materials may be better detersive surfactants than their C₁₂-C₁₄ counterparts. Accordingly, the formulator may wish to balance ease-of-manufacture *vs.* performance when selecting a particular polyhydroxy fatty acid amide for use in a given formulation.

It will also be appreciated that the solubility of the polyhydroxy fatty acid amides can be increased by having points of unsaturation and/or chain branching in the fatty acid moiety. Thus, materials such as the polyhydroxy fatty acid amides derived from oleic acid and iso-stearic acid are more soluble than their n-alkyl counterparts.

Likewise, the solubility of polyhydroxy fatty acid amides prepared from disaccharides, trisaccharides, etc., will ordinarily be greater than the solubility of their monosaccharide-derived counterpart materials. This higher solubility can be of particular assistance when formulating liquid compositions. Moreover, the polyhydroxy fatty acid amides wherein the polyhydroxy group is derived from maltose appear to function especially well as detergents when used in combination with conventional alkylbenzene sulfonate ("LAS") surfactants. While not intending to be limited by theory, it appears that the combination of LAS with the polyhydroxy fatty acid amides derived from the higher saccharides such as maltose causes a substantial and unexpected lowering of interfacial tension in aqueous media, thereby enhancing net detergency performance. (The manufacture of a polyhydroxy fatty acid amide derived from maltose is described hereinafter.)

The polyhydroxy fatty acid amides can be manufactured not only from the purified sugars, but also from hydrolyzed starches, e.g., corn starch, potato starch, or any other convenient plant-derived starch which contains the mono-, di-, etc. saccharide desired by the formulator. This is of particular importance from the economic standpoint. Thus, "high glucose" corn syrup, "high maltose" corn syrup, etc. can conveniently and economically be used. De-lignified, hydrolyzed cellulose pulp can also provide a raw material source for the polyhydroxy fatty acid amides.

As noted above, polyhydroxy fatty acid amides derived from the higher saccharides, such as maltose, lactose, etc., are more soluble than their glucose counterparts. Moreover, it appears that the more soluble polyhydroxy fatty acid amides can help solubilize their less soluble counterparts, to varying degrees. Accordingly, the formulator may elect to use a raw material comprising a high glucose corn syrup, for example, but to select a syrup which contains a modicum of maltose (e.g., 1% or more). The resulting mixture of polyhydroxy fatty acids will, in general, exhibit more preferred solubility properties over a broader range of temperatures and concentrations than would a "pure" glucose-derived polyhydroxy fatty acid amide. Thus, in addition to any economic advantages for using sugar mixtures rather than pure sugar reactants, the polyhydroxy fatty acid amides prepared from mixed sugars can offer very substantial advantages with respect to performance and/or ease-of-formulation. In some instances, however, some loss of grease removal performance (dishwashing) may be noted at fatty acid maltamide levels above about 25% and some loss in sudsing above about 33% (said percentages being the percentage of maltamide-derived polyhydroxy fatty acid amide vs. glucose-derived polyhydroxy fatty acid amide in the mixture). This can vary somewhat, depending on the chain length of the fatty acid moiety. Typically, then, the formulator electing to use such mixtures may find it advantageous to select polyhydroxy fatty acid amide mixtures which contain ratios of monosaccharides (e.g., glucose) to di- and higher saccharides (e.g., maltose) from about 4:1 to about 99:1.

The manufacture of preferred, uncyclized polyhydroxy fatty acid amides from fatty esters and N-alkyl polyols can be carried out in alcohol solvents at temperatures from about 30°C-90°C, preferably about 50°C-80°C. It has now been determined that it may be convenient for the formulator of, for example, liquid detergents to conduct such processes in 1,2-propylene glycol solvent, since the glycol solvent need not be completely removed from the reaction product prior to use in the finished detergent formulation. Likewise, the formulator of, for example, solid, typically granular, detergent compositions may find it convenient to run the process at 30°C-90°C in solvents which comprise ethoxylated alcohols, such as the ethoxylated (E0 3-8) C₁₂-C₁₄ alcohols, such as those available as NEODOL 23 E06.5 (Shell). When such ethoxylates are used, it is preferred that they not contain substantial amounts of unethoxylated alcohol and, most preferably, not contain substantial amounts of mono-ethoxylated alcohol. ("T" designation.)

While methods for making polyhydroxy fatty acid amides *per se* form no part of the invention herein, the formulator can also note other syntheses of polyhydroxy fatty acid amides as described hereinafter.

Typically, the industrial scale reaction sequence for preparing the preferred acyclic polyhydroxy fatty acid amides will comprise: Step 1 - preparing the N-alkyl polyhydroxy amine derivative from the desired sugar or sugar mixture by formation of an adduct of the N-alkyl amine and the sugar, followed by reaction with hydrogen in the presence of a catalyst; followed by Step 2 - reacting the aforesaid polyhydroxy amine with, preferably, a fatty ester to form an amide bond. While a variety of N-alkyl polyhydroxy amines useful in Step 2 of the reaction sequence can be prepared by various art-disclosed processes, the following process is convenient and makes use of economical sugar syrup as the raw material. It is to be understood that, for best results when using such syrup raw materials, the manufacturer should select syrups that are quite light in color or, preferably, nearly colorless ("water-white").

### Preparation of N-Alkyl Polyhydroxy Amine From Plant-Derived Sugar Syrup

I. Adduct Formation - The following is a standard process in which about 420 g of about 55% glucose solution (corn syrup - about 231 g glucose - about 1.28 moles) having a Gardner Color of less than 1 is reacted with about 119 g of about 50% aqueous methylamine (59.5 g of methylamine - 1.92 moles) solution. The methylamine (MMA) solution is purged and shielded with N₂ and cooled to about 10°C, or less. The corn syrup is purged and shielded with N₂ at a temperature of about 10°-20°C. The corn syrup is added slowly to the MMA solution at the indicated reaction temperature as shown. The Gardner Color is measured at the indicated approximate times in minutes.

**TABLE 1**

| Time in Minutes: | 10 | 30 | 60 | 120 | 180 | 240 |
|---|---|---|---|---|---|---|
| Reaction Temp. °C | Gardner Color (Approximate) | | | | | |
| 0 | 1 | 1 | 1 | 1 | 1 | 1 |
| 20 | 1 | 1 | 1 | 1 | 1 | 1 |
| 30 | 1 | 1 | 2 | 2 | 4 | 5 |
| 50 | 4 | 6 | 10 | - | - | - |

As can be seen from the above data, the Gardner Color for the adduct is much worse as the temperature is raised above about 30°C and at about 50°C, the time that the adduct has a Gardner Color below 7 is only about 30 minutes. For longer reaction, and/or holding times, the temperature should be less than about 20°C. The Gardner Color should be less than about 7, and preferably less than about 4 for good color glucamine.

When one uses lower temperatures for forming the adduct, the time to reach substantial equilibrium concentration of the adduct is shortened by the use of higher ratios of amine to sugar. With the 1.5:1 mole ratio of amine to sugar noted, equilibrium is reached in about two hours at a reaction temperature of about 30°C. At a 1.2:1 mole ratio, under the same conditions, the time is at least about three hours. For good color, the combination of amine:sugar ratio; reaction temperature; and reaction time is selected to achieve substantially equilibrium conversion, e.g., more than about 90%, preferably more than about 95%, even more preferably more than about 99%, based upon the sugar, and a color that is less than about 7, preferably less than about 4, more preferably less than about 1, for the adduct.

Using the above process at a reaction temperature of less than about 20°C and corn syrups with different Gardner Colors as indicated, the MMA adduct color (after substantial equilibrium is reached in at least about two hours) is as indicated.

**TABLE 2**

| | Gardner Color (Approximate) | | | | | | |
|---|---|---|---|---|---|---|---|
| Corn syrup | 1 | 1 | 1 | 1+ | 0 | 0 | 0+ |
| Adduct | 3 | 4/5 | 7/8 | 7/8 | 1 | 2 | 1 |

As can be seen from the above, the starting sugar material must be very near colorless in order to consistently have adduct that is acceptable. When the sugar has a Gardner Color of about 1, the adduct is sometimes acceptable and sometimes not acceptable. When the Gardner Color is above 1 the resulting adduct is unacceptable. The better the initial color of the sugar, the better is the color of the adduct.

II. Hydrogen Reaction - Adduct from the above having a Gardner Color of 1 or less is hydrogenated according to the following procedure.

About 539 g of adduct in water and about 23.1 g of United Catalyst G49B Ni catalyst are added to a one liter autoclave and purged two times with 200 psig H₂ at about 20°C. The H₂ pressure is raised to about 1400 psi and the temperature is raised to about 50°C. The pressure is then raised to about 1600 psig and the temperature is held at about 50-55°C for about three hours. The product is about 95% hydrogenated at this point. The temperature is then raised to about 85°C for about 30 minutes and the reaction mixture is decanted and the catalyst is filtered out. The product, after removal of water and MMA by evaporation, is about 95% N-methyl glucamine, a white powder.

The above procedure is repeated with about 23.1 g of Raney Ni catalyst with the following changes. The catalyst is washed three times and the reactor, with the catalyst in the reactor, is purged twice with 200 psig H₂ and the reactor is pressurized with H₂ at 1600 psig for two hours, the pressure is released at one hour and the reactor is repressurized to 1600 psig. The adduct is then pumped into the reactor which is at 200 psig and 20°C, and the reactor is purged with 200 psig H₂, etc., as above.

The resulting product in each case is greater than about 95% N-methyl glucamine; has less than about 10 ppm Ni based upon the glucamine; and has a solution color of less than about Gardner 2.

The crude N-methyl glucamine is color stable to about 140°C for a short exposure time.

It is important to have good adduct that has low sugar content (less than about 5%, preferably less than about 1%) and a good color (less than about 7, preferably less than about 4 Gardner, more preferably less than about 1).

In another reaction, adduct is prepared starting with about 159 g of about 50% methylamine in water, which is purged and shielded with N₂ at about 10-20°C. About 330 g of about 70% corn syrup (near water-white) is degassed with N₂ at about 50°C and is added slowly to the methylamine solution at a temperature of less than about 20°C. The solution is mixed for about 30 minutes to give about 95% adduct that is a very light yellow solution.

About 190 g of adduct in water and about 9 g of United Catalyst G49B Ni catalyst are added to a 200 ml autoclave and purged three times with H₂ at about 20°C. The H₂ pressure is raised to about 200 psi and the temperature is raised to about 50°C. The pressure is raised to 250 psi and the temperature is held at about 50-55°C for about three hours. The product, which is about 95% hydrogenated at this point, is then raised to a temperature of about 85°C for about 30 minutes and the product, after removal of water and evaporation, is about 95% N-methyl glucamine, a white powder.

It is also important to minimize contact between adduct and catalyst when the H₂ pressure is less than about 1000 psig to minimize Ni content in the glucamine. The nickel content in the N-methyl glucamine in this reaction is about 100 ppm as compared to the less than 10 ppm in the previous reaction.

The following reactions with H₂ are run for direct comparison of reaction temperature effects.

A 200 ml autoclave reactor is used following typical procedures similar to those set forth above to make adduct and to run the hydrogen reaction at various temperatures.

Adduct for use in making glucamine is prepared by combining about 420 g of about 55% glucose (corn syrup) solution (231 g glucose; 1.28 moles) (the solution is made using 99DE corn syrup from CarGill, the solution having a color less than Gardner 1) and about 119 g of 50% methylamine (59.5 g MMA; 1.92 moles) (from Air Products).

The reaction procedure is as follows:
1. Add about 119 g of the 50% methylamine solution to a N₂ purged reactor, shield with N₂ and cool down to less than about 10°C.
2. Degas and/or purge the 55% corn syrup solution at 10-20°C with N₂ to remove oxygen in the solution.
3. Slowly add the corn syrup solution to the methylamine solution and keep the temperature less than about 20°C.
4. Once all corn syrup solution is added in, agitate for about 1-2 hours.

The adduct is used for the hydrogen reaction right after making, or is stored at low temperature to prevent further degradation.

The glucamine adduct hydrogen reactions are as follows:
1. Add about 134 g adduct (color less than about Gardner 1) and about 5.8 g G49B Ni to a 200 ml autoclave.
2. Purge the reaction mix with about 200 psi H₂ twice at about 20-30°C.
3. Pressure with H₂ to about 400 psi and raise the temperature to about 50°C.
4. Raise pressure to about 500 psi, react for about 3 hours. Keep temperature at about 50-55°C. Take Sample 1.
5. Raise temperature to about 85°C for about 30 minutes.
6. Decant and filter out the Ni catalyst. Take Sample 2. Conditions for constant temperature reactions:
1. Add about 134 g adduct and about 5.8 g G49B Ni to a 200 ml autoclave.
2. Purge with about 200 psi H₂ twice at low temperature.
3. Pressure with H₂ to about 400 psi and raise temperature to about 50°C.
4. Raise pressure to about 500 psi, react for about 3.5 hours. Keep temperature at indicated temperature.
5. Decant and filter out the Ni catalyst. Sample 3 is for about 50-55°C; Sample 4 is for about 75°C; and Sample 5 is for about 85°C. (The reaction time for about 85°C is about 45 minutes.)

All runs give similar purity of N-methyl glucamine (about 94%); the Gardner Colors of the runs are similar right after reaction, but only the two-stage heat treatment gives good color stability; and the 85°C run gives marginal color immediately after reaction.

### Preparation of Maltamine Amide

The preparation of the tallow (hardened) fatty acid amide of N-methyl maltamine for use in detergent compositions according to this invention is as follows.

Step 1 - Reactants: Maltose monohydrate (Aldrich, lot 01318KW); methylamine (40 wt% in water) (Aldrich, lot 03325TM); Raney nickel, 50% slurry (UAD 52-73D, Aldrich, lot 12921LW).

The reactants are added to glass liner (250 g maltose, 428 g methylamine solution, 100 g catalyst slurry - 50 g Raney Ni) and placed in 3 L rocking autoclave, which is purged with nitrogen (3X500 psig) and hydrogen (2X500 psig) and rocked under H₂ at room temperature over a weekend at temperatures ranging from 28°C to 50°C. The crude reaction mixture is vacuum filtered 2X through a glass microfiber filter with a silica gel plug. The filtrate is concentrated to a viscous material. The final traces of water are azetroped off by dissolving the material in methanol and then removing the methanol/water on a rotary evaporator. Final drying is done under high vacuum. The crude product is dissolved in refluxing methanol, filtered, cooled to recrystallize, filtered and the filter cake is dried under vacuum at 35°C. This is cut #1. The filtrate is concentrated until a precipitate begins to form and is stored in a refrigerator overnight. The solid is filtered and dried under vacuum. This is cut #2. The filtrate is again concentrated to half its volume and a recrystallization is performed. Very little precipitate forms. A small quantity of ethanol is added and the solution is left in the freezer over a weekend. The solid material is filtered and dried under vacuum. The combined solids comprise N-methyl maltamine which is used in Step 2 of the overall synthesis.

Step 2 - Reactants: N-methyl maltamine (from Step 1); hardened tallow methyl esters; sodium methoxide (25% in methanol); absolute methanol (solvent); mole ratio 1:1 amine:ester; initial catalyst level 10 mole % (w/r maltamine), raised to 20 mole %; solvent level 50% (wt.).

In a sealed bottle, 20.36 g of the tallow methyl ester is heated to its melting point (water bath) and loaded into a 250 ml 3-neck round-bottom flask with mechanical stirring. The flask is heated to *ca.* 70°C to prevent the ester from solidifying. Separately, 25.0 g of N-methyl maltamine is combined with 45.36 g of methanol, and the resulting slurry is added to the tallow ester with good mixing. 1.51 g of 25% sodium methoxide in methanol is added. After four hours the reaction mixture has not clarified, so an additional 10 mole % of catalyst (to a total of 20 mole %) is added and the reaction is allowed to continue overnight (*ca.* 68°C) after which time the mixture is clear. The reaction flask is then modified for distillation. The temperature is increased to 110°C. Distillation at atmospheric pressure is continued for 60 minutes. High vacuum distillation is then begun and continued for 14 minutes, at which time the product is very thick. The product is allowed to remain in the reaction flask at 110°C (external temperature) for 60 minutes. The product is scraped from the flask and triturated in ethyl ether over a weekend. Ether is removed on a rotary evaporator and the product is stored in an oven overnight, and ground to a powder. Any remaining N-methyl maltamine is removed from the product using silica gel. A silica gel slurry in 100% methanol is loaded into a funnel and washed several times with 100% methanol. A concentrated sample of the product (20 g in 100 ml of 100% methanol) is loaded onto the silica gel and eluted several times using vacuum and several methanol washes. The collected eluant is evaporated to dryness (rotary evaporator). Any remaining tallow ester is removed by trituration in ethyl acetate overnight, followed by filtration. The filter cake is then vacuum dried. The product is the tallow-alkyl N-methyl maltamide.

In an alternate mode, Step 1 of the foregoing reaction sequence can be conducted using commercial corn syrup comprising glucose or mixtures of glucose and, typically, 5%, or higher, maltose. The resulting polyhydroxy fatty acid amides and mixtures can be used in any of the detergent compositions herein.

In still another mode, Step 2 of the foregoing reaction sequence can be carried out in 1,2-propylene glycol or NEODOL. At the discretion of the formulator, the propylene glycol or NEODOL need not be removed from the reaction product prior to its use to formulate detergent compositions. Again, according to the desires of the formulator, the methoxide catalyst can be neutralized by citric acid to provide sodium citrate, which can remain in the polyhydroxy fatty acid amide.

Depending on the desires of the formulator, the compositions herein can contain more or less of various suds control agents. Typically, for dishwashing high sudsing is desirable so no suds control agent will be used. For fabric laundering in top-loading washing machines some control of suds may be desirable, and for front-loaders some considerable degree of suds control may be preferred. A wide variety of suds control agents are known in the art and can be routinely selected for use herein. Indeed, the selection of suds control agent, or mixtures of suds control agents, for any specific detergent composition will depend not only on the presence and amount of polyhydroxy fatty acid amide used therein, but also on the other surfactants present in the formulation. However, it appears that, for use with polyhydroxy fatty acid amides, silicone-based suds control agents of various types are more efficient (i.e., lower levels can be used) than various other types of suds control agents. The silicone suds control agents available as AE, X2-3419, Q2-3302 and DC-544 (Dow Corning) are particularly useful.

### Fatty Acids

For compositions where especially high sudsing is desired (e.g., dishwashing), it is preferred that less than about 5%, preferably less than about 2%, most preferably no C₁₄ or higher fatty acids be present, since these can suppress sudsing. Liquid detergent compositions herein are preferably substantially free of a suds-suppressing amount of C₁₄ and higher fatty acid. Accordingly, the formulator of high sudsing compositions will desirably avoid the introduction of suds-suppressing amounts of such fatty acids into high sudsing compositions with the polyhydroxy fatty acid amide, and/or avoid the formation of C₁₄ and higher fatty acids on storage of the finished compositions. One simple means is to use C₁₂ ester reactants to prepare the polyhydroxy fatty acid amides herein. Fortunately, the use of amine oxide or sulfobetaine surfactants can overcome some of the negative sudsing effects caused by the fatty acids. In fact, any fatty acids should be avoided (less than about 2.5% by weight is preferred) because of potential precipitation problems.

The formulator wishing to add anionic optical brighteners to liquid detergents containing relatively high concentrations (e.g., 10% and greater) of anionic or polyanionic substituents such as the polycarboxylate builders may find it useful to pre-mix the brightener with water and the polyhydroxy fatty acid amide, and then to add the pre-mix to the final composition.

It will be appreciated by those skilled in the chemical arts that the preparation of the polyhydroxy fatty acid amides herein using the di- and higher saccharides such as maltose will result in the formation of polyhydroxy fatty acid amides wherein linear substituent Z is "capped" by a polyhydroxy ring structure. Such materials are fully contemplated for use herein and do not depart from the spirit and scope of the invention as disclosed and claimed.

### C. Calcium

From about 0.1% to about 3%, more preferably from about 0.2% to about 2%, most preferably from about 0.3% to about 1.5%, of calcium ions are included in the detergent compositions herein. It has been found for compositions containing the present polyhydroxy fatty acid amide that the presence of calcium greatly improves the cleaning of greasy soils. This is especially true when the compositions are used in softened water, which contains few divalent ions.

Preferably, the calcium ions are added as a chloride, hydroxide, oxide, acetate, or nitrate salt to compositions containing an alkali metal or ammonium salt of the anionic sulfate, most preferably the ammonium salt (see methods of incorporation in Section E below). The calcium salts are preferably soluble.

The calcium ions may be present in the composition as salts.

The amount of calcium ions present in compositions of the invention may be dependent upon the amount of total anionic surfactant present therein. The molar ratio of calcium ions to total anionic surfactant is preferably from about 0.25:1 to about 1:2 for compositions of the invention.

Boric acid is preferably not used in these detergent compositions.

The detergent compositions herein, especially liquid detergent compositions, more especially light duty liquids, are substantially free of chelating agent having a log stability constant above about 8. Strong chelating agents are not necessary for stable liquid formulations. Citrate, tripolyphosphate, and carbonate are preferably included in the detergent compositions herein. Strong chelating agents with log stability constants between about 9 and 12 such as ethylenediamine tetraacetate and diethylenetriaminepentaacetate, are not desirable in the compositions herein, especially in light duty liquids.

### D. Lime Soap Dispersing Agent

The compositions of the present invention preferably comprise from about 0.2% to about 20%, more preferably from about 0.5% to about 10%, most preferably from about 1% to about 6%, by weight of lime soap dispersing agent ("LSDA"), particularly when C₁₀-C₁₆ alkyl ethoxy (E_{0.5}-E_{2.5}) sulfate is employed in the composition.

A measure of the dispersibility of an LSDA is the lime soap dispersant requirement ("LSDR"). "The LSDR is the minimum number of grams of the test LSDA which will just prevent a solution of 100g. of sodium oleate in 320 ppm hard water from precipitating." N. Linfield, Surfactant Science Series, vol. 7, p.3. A low LSDR means that the LSDA is a good dispersant. Detergency is independent of lime soap dispersibility. Linfield, p.2.

LSDRs of various LSDAs are listed below.

**TABLE 1**

| Item | LSDA, Formula | LSDR |
|---|---|---|
| A | RCOOCH₂CH₂SO₃Na | 10 |
| B | RCH(SO₃Na)COOCH₃, TMS | 8 |
| C | RCH(SO₃Na)COOCH₂CH₂SO₃Na | 5 |
| D | RCON(CH₃)CH₂CH₂SO₃Na, IgT | 6 |
| E | RO(CH₂CH₂O)SO₃Na | 4 |
| F | RCONHCH₂CH(CH₃)OSO₃Na, TAM | 4 |
| G | R'C₆H₄SO₂NHCH₂CH₂OSO₃Na | 6 |
| H | R'C₆H₄COCH(SO₃Na)CH₂COOCH₃ | 8 |
| I | C₉H₁₉C₆H₄(OCH₂CH₂)_{9.5} OH | 5 |
| J | RCONH(CH₂CH₂O)₁₅ | 3 |
| K | RN⁺(CH₃)₂CH₂CH₂CH₂SO₃⁻ | 3 |
| L | RCONHCH₂CH₂CH₂N⁺(CH₃)₂CH₂CH₂CH₂SO₃⁻ | 3 |
| M | R'C₆H₄SO₃Na* | 40 |

| | | |
|---|---|---|
| R represents an alkyl group derived from tallow and R' represents an alkyl group in the C₁₁-C₁₃ range corresponding to commercial detergent alkylates. * LAS N. Linfield, Surfactant Science Series, vol. 7, p. 4. | | |

Preferred LSDAs for use herein are A-D and F-L from Table 1. More preferred LSDAs are K and L. More preferred are C₈-C₂₂ sulfobetaine and/or hydroxysulfobetaine. Most preferred are C₁₂-C₁₈ sulfobetaine and/or hydroxysulfobetaine. Other preferred LSDAs include sodium or potassium polyacrylate, polymaleate/acrylate copolymer, and betaine, especially alkyl dimethyl betaine. LSDAs with an LSDR of between about 1 and about 20, most preferably between about 2 and about 10, are preferred.

It is believed that alkyl ethoxy sulfate with greater than about 2.5 moles of ethylene oxide is its own lime soap dispersing agent; the ethylene oxide acts as a solubilizer. See LSDA "E" above.

Linear alkylbenzene sulfonate ("LAS") and other anionic sulfonates are preferably avoided herein where a stable product is desired because of their tendency to rapidly bind with calcium and form precipitates. LAS has an LSDR of 40 (a poor lime soap dispersant).

### E. Magnesium

From about 0.05% to about 1.5%, most preferably from about 0.3% to about 0.9%, by weight, of magnesium ions are preferably added to the liquid detergent compositions of the invention for improved product stability, as well as improved sudsing and skin mildness.

The technique of incorporating the calcium (and magnesium) into the compositions of the present invention is not thought to be critical and can be accomplished in a number of ways.

First, individual anionic surfactants can be made as aqueous solutions of alkali metal or ammonium salts which may be mixed together with a hydrotrope, after which the calcium and optionally magnesium can be introduced as a water soluble salt, such as chloride (preferred), hydroxide, oxide, acetate, and/or nitrate. Optional minor ingredients may then be added before pH and viscosity are adjusted. The pH is preferably adjusted before the addition of the calcium salt. This preferred method has the advantage of utilizing conventional techniques and equipment, although it does result in the introduction of additional chloride ions.

The preferred calcium ion:magnesium ion ratio is between about 1:10 and about 1:2, preferably between about 1:4 and about 1:2. It is preferred that the calcium ions are introduced by adding calcium chloride dihydrate to the composition and that the magnesium ions are introduced by adding magnesium chloride hexahydrate to the composition. From about 1% to about 5% by weight of calcium chloride dihydrate, and optionally from about 3% to about 7% of magnesium chloride hexahydrate, are preferred for a light duty liquid composition herein.

If the anionic surfactants are in the acid form, then the magnesium can be added by a second method: neutralization of the acid with a magnesium oxide or magnesium hydroxide slurry in water. Calcium can be treated similarly. The use of calcium hydroxide is preferred. This technique avoids the addition of chloride ions, which improves chill point and reduces corrosive properties. The neutralized surfactant salts and the hydrotrope are then added to the final mixing tank and any optional ingredients are added before adjusting the pH.

A third technique for incorporating calcium into granular detergent compositions is to add calcium salt, preferably calcium sulfate, into the crutcher prior to spray drying.

### F. Composition pH

The detergent compositions herein preferably have a pH in a 10% solution in water at 20°C of between about 5.5 and about 11.0. Granular detergent compositions herein preferably have a pH of between about 9.5 and about 11.0.

The liquid detergent compositions hereof will preferably be formulated such that during use in aqueous cleaning operations, the wash water will have a pH of between about 5.0 and about 8.0. The liquid compositions themselves preferably have a pH in a 10% solution water at 20°C of between about 5.5 and about 8.5, most preferably between about 6.8 and about 7.8.

Techniques for controlling pH at recommended usage levels include the use of buffers, alkali, acids, etc., and are well known to those skilled in the art. Dilute hydrochloric acid or citric acid is preferred for pH adjustment.

### G. Liquid or Gel Compositions

### Liquid Carrier

In a preferred embodiment, the detergent compositions of the present invention are liquid detergent compositions. These preferred liquid detergent compositions comprise from about 94% to about 35% by weight, preferably from about 90% to about 50% by weight, most preferably from about 80% to about 60% by weight of a liquid carrier, e.g., water, preferably a mixture of water and a C₁-C₄ monohydric alcohol (e.g., ethanol, propanol, isopropanol, butanol, and mixtures thereof), with ethanol being the preferred alcohol. A preferred way to make light duty liquids of the present invention is to combine the polyhydroxy fatty acid amide and the alkyl (ethoxy) sulfate with water and ethanol. pH is adjusted and then calcium and optionally magnesium ions are mixed into the composition as aqueous solutions of chloride salts. The mixture is blended and hydrotrope may be added to adjust the viscosity. Perfume, dye, opacifier, and other optional ingredients may then be added.

### Gel Thickening Agent

The detergent compositions of the present invention may also be in the form of a gel. Such compositions are typically formulated in polyalkenyl polyether and having a molecular weight of from about 750,000 to about 4,000,000.

Highly preferred examples of these polycarboxylate polymer thickeners are the Carbopol 600 series resins available from B. F. Goodrich. Especially preferred are Carbopol 616 and 617. It is believed that these resins are more highly cross-linked than the 900 series resins and have molecular weights between about 1,000,000 and 4,000,000. Mixtures of polycarboxylate polymers as herein described may also be used in the present invention. Particularly preferred is a mixture of Carbopol 616 and 617 series resins.

The polycarboxylate polymer thickener is utilized preferably with essentially no clay thickening agents. In fact, it has been found that if the polycarboxylate polymers of the present invention are utilized with clay in the composition of the present invention, a less desirable product, in terms of phase instability, results. In other words, the polycarboxylate polymer is preferably used instead of clay as a thickening/stabilizing agent in the present compositions.

If the polycarboxylate polymer is used as a thickening agent in the compositions of the present invention, it is typically present at a level of from about 0.1% to about 10%, preferably from about 0.2% to about 2% by weight.

The thickening agents are preferably used to provide a yield value of from about 50 to about 350 and most preferably from about 75 to about 250. The yield value is an indication of the shear stress at which the gel strength is exceeded and flow is initiated. It is measured herein with a Brookfield RVT model viscometer with a T-bar B spindle at 25°C utilizing a Helipath drive upward during associated readings. The system is set to 0.5 rpm and a reading is taken for the composition to be tested after 30 seconds or after the system is stable. The system is stopped and the rpm is reset to 1.0 rpm. A reading is taken for the same composition after 30 seconds or after the system is stable. Stress at zero shear is equal to two times the 0.5 rpm reading minus the reading at 1.0 rpm. The yield value is calculated as the stress at zero shear times 18.8 (conversion factor).

### H. Other Optional Components

### Other Anionic Surfactants

Other anionic surfactants useful for detersive purposes can also be included in the compositions hereof. Exemplary, non-limiting useful anionics include salts (e.g., sodium, potassium, ammonium, and substituted ammonium salts such as mono-, di- and triethanolamine salts) of soap, C₈-C₂₂ alkylsulfates, C₈-C₂₄ alkylpolyethersulfates (containing up to 10 moles of ethylene oxide); fatty acyl glycerol sulfates, alkyl phenol ethylene oxide ether sulfates, alkyl phosphates, isethionates such as the acyl isethionates, acyl taurates, fatty acid amides, alkyl succinates and sulfosuccinates, acyl sarcosinates, sulfates of alkylpolysaccharides such as the sulfates of alkylpolyglucoside, alkyl ether carbonates, alkyl ethoxy carboxylates, fatty acids esterified with isethionic acid and neutralized with sodium hydroxide, and fatty acids amides of methyl tauride. Further examples are described in "Surface Active Agents and Detergents" (Vol. I and II by Schwartz, Perry and Berch). A variety of such surfactants are also generally disclosed in U.S. Patent 3,929,678, issued December 30, 1975 to Laughlin, et al. at Column 23, line 58 through Column 29, line 23.

### Nonionic Surfactants

Suitable nonionic detergent surfactants are generally disclosed in U.S. Patent 3,929,678, Laughlin et al., issued December 30, 1975, at column 13, line 14 through column 16, line 6. Exemplary, non-limiting classes of useful nonionic surfactants are listed below.
1. The polyethylene, polypropylene, and polybutylene oxide condensates of alkyl phenols. In general, the polyethylene oxide condensates are preferred. These compounds include the condensation products of alkyl phenols having an alkyl -group containing from 6 to 12 carbon atoms in either a straight- or branched-chain configuration with the alkylene oxide. Commercially available nonionic surfactants of this type include Igepal™ CO-630, marketed by the GAF Corporation; and Triton^{TM} X-45, X-114, X-100, and X-102, all marketed by the Rohm & Haas Company.
2. The condensation products of aliphatic alcohols with from about 1 to about 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from about 10 to about 20 carbon atoms with from about 2 to about 10 moles of ethylene oxide per mole of alcohol.
3. The condensation products of ethylene oxide with a hydrophobic base formed by the condensation of propylene oxide with propylene glycol. The hydrophobic portion of these compounds preferably has a molecular weight of from about 1500 to about 1800 and exhibits water insolubility.
4. The condensation products of ethylene oxide with the product resulting from the reaction of propylene oxide and ethylenediamine.
5. Semi-polar nonionic surfactants are a special category of nonionic surfactants which include water-soluble amine oxides containing one alkyl moiety of from 10 to 18 carbon atoms and 2 moieties selected from the group consisting of alkyl groups and hydroxyalkyl groups containing from 1 to 3 carbon atoms; water-soluble phosphine oxides containing one alkyl moiety of from 10 to 18 carbon atoms and 2 moieties selected from the group consisting of alkyl groups and hydroxyalkyl groups containing from 1 to 3 carbon atoms; and water-soluble sulfoxides containing one alkyl moiety of from 10 to 18 carbon atoms and a moiety selected from the group consisting of alkyl and hydroxyalkyl moieties of from 1 to 3 carbon atoms. Semi-polar nonionic detergent surfactants include the amine oxide surfactants
6. Alkylpolysaccharides disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e.g., a polyglycoside, hydrophilic group containing from about 1.3 to about 10, preferably from about 1.3 to about 3, most preferably from about 1.3 to about 2.7 saccharide units.
7. Fatty acid amide surfactants having the formula: 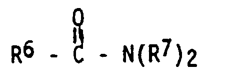 wherein R⁶ is an alkyl group containing from 7 to 21, preferably from 9 to 17, carbon atoms and each R⁷ is selected from the group consisting of hydrogen, C₁-C₄ alkyl, C₁-C₄ hydroxyalkyl, and -(C₂H₄O)ₓH where x varies from about 1 to about 3.

### Other Surfactants

Ampholytic surfactants may also be incorporated into the detergent compositions hereof. These surfactants can be broadly described as aliphatic derivatives of secondary or tertiary amines, or aliphatic derivatives of heterocyclic secondary and tertiary amines in which the aliphatic radical can be straight-branched chains. One of the aliphatic substituents contains at least 8 carbon atoms, typically from 8 to 18 carbon atoms, and at least one contains an anionic water-solubilizing group, e.g., carboxy, sulfonate, sulfate. See U.S. Patent No. 3,929,678 to Laughlin et al., issued December 30, 1975, at column 19, lines 18-35 for examples of useful ampholytic surfactants.

Zwitterionic surfactants may also be incorporated into the detergent compositions hereof. These surfactants can be broadly described as derivatives of secondary and tertiary amines, derivatives of heterocyclic secondary and tertiary amines, or derivatives of quaternary ammonium, quaternary phosphonium or tertiary sulfonium compounds. See U.S. Patent No. 3,929,678 to Laughlin et al., issued December 30, 1975, at column 19, line 38 through column 22, line 48 for examples of useful zwitterionic surfactants.

Such ampholytic and zwitterionic surfactants are generally used in combination with one or more anionic and/or nonionic surfactants.

If included in the compositions of the present invention, these optional additional surfactants are typically present at a concentration of from about 1% to about 15%, preferably from about 2% to about 10% by weight.

### Detergency Builders

Other optional ingredients include detergency builders, either of the organic or inorganic type. Granular detergent compositions herein preferably comprise from about 5% to about 50% by weight of detergency builder, most preferably citrate and carbonate. Detergency builder levels higher than about 50% are not desirable herein.

Examples of water-soluble inorganic builders which can be used, either alone or in admixture with themselves or with organic alkaline sequestrant builder salts, are glycine, alkyl and alkenyl succinates, alkali metal carbonates, phosphates, polyphosphates, and silicates. Specific examples of such salts are sodium tripolyphosphate, sodium carbonate, potassium carbonate, sodium pyrophosphate, potassium pyrophosphate, potassium tripolyphosphate, and sodium hexametaphosphate. Examples of organic builder salts which can be used alone, or in admixture with each other, or with the preceding inorganic alkaline builder salts, are alkali metal polycarboxylates, examples of which include, but are not limited to, water-soluble citrates such as sodium and potassium citrate, sodium and potassium tartrate, sodium and potassium nitrilo triacetates, sodium and potassium N-2(2-hydroxyethyl)-nitrilo diacetates, sodium and potassium oxydisuccinates, and sodium and potassium tartrate mono- and di-succinates, such as those described in U.S. Patent 4,663,071 (Bush et al., issued May 5, 1987), the disclosure of which is incorporated herein. Other organic detergency builders, such as water-soluble phosphonates, can be used in the compositions of the present invention.

Detergency builders in general have limited value when the compositions of the present invention are in the form of light duty liquid dishwashing detergent compositions. If included in light duty liquids, these optional builders are typically present at a concentration of from about 1.0% to about 10%, preferably from about 2% to about 5%, by weight.

### Other Ingredients

Other desirable ingredients include diluents, solvents, dyes, perfumes, opacifiers (preferred), and hydrotropes (preferred). Diluents can be inorganic salts, such as sodium and potassium sulfate, ammonium chloride, sodium and potassium chloride, sodium bicarbonate, etc. Diluents useful in the compositions of the present invention are typically present at levels of from about 1% to about 10%, preferably from about 2% to about 5% by weight.

Solvents useful herein include water and lower molecular weight alcohols, such as ethyl alcohol, isopropyl alcohol, etc. Solvents useful in the compositions of the present invention are typically present at levels of from about 1% to about 60%, preferably from about 5% to about 50% by weight.

Hydrotropes such as sodium, potassium, and ammonium xylene sulfonate (preferred), sodium, potassium and ammonium toluene sulfonate, sodium, potassium and ammonium cumene sulfonate, and mixtures thereof, and related compounds (as disclosed in U.S. Patent 3,915,903) can be utilized in the interests of achieving a desired product phase stability and viscosity. Hydrotropes useful in the compositions of the present invention are typically present at levels of from about 1% to about 10%, preferably from about 2% to about 5%, by weight.

Optional ingredients useful when the compositions of the present invention are used in liquid dishwashing detergent applications include drainage promoting ethoxylated nonionic surfactants of the type disclosed in U.S. Patent 4,316,824, issued Pancheri, issued February 23, 1982.

The claimed compositions of the present invention are beneficial in that they provide unexpectedly improved grease cleaning performance and clean dishes without imparting a "greasy" feel to the cleaned dish.

Opacifiers such as Lytron (Morton Thiokol, Inc.), a modified polystyrene latex, or ethylene glycol distearate can be added, preferably as a last step. Lytron can be aded directly as a dispersion with mixing. Ethylene glycol distearate can be added in a molten state with rapid mixing to form pearlescent crystals. Opacifiers useful herein, particularly for light duty liquids, are typically present at levels of from about 0.2% to about 10%, preferably from about 0.5% to about 6% by weight.

### I. Method Aspect

In the method aspect of this invention, soiled dishes are contacted with an effective amount, typically from about 0.5 ml. to about 20 ml. (per 25 dishes being treated), preferably from about 3 ml. to about 10 ml., of the detergent composition of the present invention. The actual amount of liquid detergent composition used will be based on the judgement of user, and will typically depend upon factors such as the particular product formulation of the composition, including the concentration of active ingredient in the composition, the number of soiled dishes to be cleaned, the degree of soiling on the dishes, and the like. The particular product formulation, in turn, will depend upon a number of factors, such as the intended market (i.e., U.S., Europe, Japan, etc.) for the composition product. The following are examples of typical methods in which the detergent compositions of the present invention may be used to clean dishes. These examples are for illustrative purposes and are not intended to be limiting.

In a typical U.S. application, from about 3 ml. to about 15 ml., preferably from about 5 ml. to about 10 ml. of a liquid detergent composition is combined with from about 1,000 ml. to about 10,000 ml., more typically from about 3,000 ml. to about 5,000 ml. of water in a sink having a volumetric capacity in the range of from about 5,000 ml. to about 20,000 ml., more typically from about 10,000 ml. to about 15,000 ml. The detergent composition has a surfactant mixture concentration of from about 21% to about 44% by weight, preferably from about 25% to about 40% by weight. The soiled dishes are immersed in the sink containing the detergent composition and water, where they are cleaned by contacting the soiled surface of the dish with a cloth, sponge, or similar article. The cloth, sponge, or similar article may be immersed in the detergent composition and water mixture prior to being contacted with the dish surface. and is typically contacted with the dish surface for a period of time ranging from about 1 to about 10 seconds, although the actual time will vary with each application and user. The contacting of the cloth, sponge, or similar article to the dish surface is preferably accompanied by a concurrent scrubbing of the dish surface.

In a typical European market application, from about 3 ml. to about 15 ml., preferably from about 3 ml. to about 10 ml. of a liquid detergent composition is combined with from about 1,000 ml. to about 10,000 ml., more typically from about 3,000 ml. to about 5,000 ml. of water in a sink having a volumetric capacity in the range of from about 5,000 ml. to about 20,000 ml., more typically from about 10,000 ml. to about 15,000 ml. The detergent composition has a surfactant mixture concentration of from about 20% to about 50% by weight, preferably from about 30% to about 40%, by weight. The soiled dishes are immersed in the sink containing the detergent composition and water, where they are cleaned by contacting the soiled surface of the dish with a cloth, sponge, or similar article. The cloth, sponge, or similar article may be immersed in the detergent composition and water mixture prior to being contacted with the dish surface, and is typically contacted with the dish surface for a period of time ranging from about 1 to about 10 seconds, although the actual time will vary with each application and user. The contacting of the cloth, sponge, or similar article to the dish surface is preferably accompanied by a concurrent scrubbing of the dish surface.

In a typical Latin American and Japanese market application, from about 1 ml. to about 50 ml., preferably from about 2 ml. to about 10 ml. of a detergent composition is combined with from about 50 ml. to about 2,000 ml., more typically from about 100 ml. to about 1,000 ml. of water in a bowl having a volumetric capacity in the range of from about 500 ml. to about 5,000 ml., more typically from about 500 ml. to about 2,000 ml. The detergent composition has a surfactant mixture concentration of from about 5% to about 40% by weight, preferably from about 10% to about 30% by weight. The soiled dishes are cleaned by contacting the soiled surface of the dish with a cloth, sponge, or similar article. The cloth, sponge, or similar article may be immersed in the detergent composition and water mixture prior to being contacted with the dish surface, and is typically contacted with the dish surface for a period of time ranging from about 1 to about 10 seconds, although the actual time will vary with each application and user. The contacting of the cloth, sponge, or similar article to the dish surface is preferably accompanied by a concurrent scrubbing of the dish surface.

Another method of use will comprise immersing the soiled dishes into a water bath without any liquid dishwashing detergent. A device for absorbing liquid dishwashing detergent, such as a sponge, is placed directly into a separate quantity of undiluted liquid dishwashing composition for a period of time typically ranging from about 1 to about 5 seconds. The absorbing device, and consequently the undiluted liquid dishwashing composition, is then contacted individually to the surface of each of the soiled dishes to remove said soiling. The absorbing device is typically contacted with each dish surface for a period of time range from about 1 to about 10 seconds, although the actual time of application will be dependent upon factors such as the degree of soiling of the dish. The contacting of the absorbing device to the dish surface is preferably accompanied by concurrent scrubbing.

### EXAMPLES

The following examples illustrate the compositions of the present invention, but are not necessarily meant to limit or otherwise define the scope of the invention. All parts, percentages and ratios used herein are by weight unless otherwise specified.

### EXAMPLE I

The following light duty liquid composition of the present invention is prepared according to the descriptions set forth below.

Formulation A is made initially by combining the N-methyl glucamide (a polyhydroxy fatty acid amide, with fatty acid level of less than 2%), water and ethanol at 50°C. The pH of the mixture is adjusted with dilute or concentrated hydrochloric acid before the addition of the remaining ingredients. The final pH (10% solution) is adjusted to 7.0-7.2.

Formulations B and C are made the same way except that calcium ion (added as calcium chloride dihydrate) and/or magnesium ion (added as magnesium chloride hexahydrate) are added after the initial pH adjustment.

The mixture is blended until a homogeneous, clear solution is obtained. Additional hydrotrope may be added to trim the liquid composition to the desired viscosity; ideally 50-1000 cps, as measured by a Brookfield viscometer at 70°F (21.1°C). Perfume, dye and other ingredients are added as the last step.

| | %: By Weight | | |
|---|---|---|---|
| Component | A | B | C |
| C₁₂₋₁₄ alkyl N-methyl glucamide | 12.70 | 12.70 | 12.70 |
| Ammonium C₁₂₋₁₃ alkyl ethoxy (Peaked at 2.0) sulfate | 12.70 | 12.70 | 12.70 |
| C₉₋₁₁ alkyl ethoxy (10 ave.) alcohol | 7.00 | 7.00 | 7.00 |
| Calcium chloride dihydrate | --- | --- | 3.70 |
| Magnesium chloride hexahydrate | --- | 5.53 | --- |
| Ethanol | 5.00 | 5.00 | 5.00 |
| Sodium toluene sulfonate | 2.00 | 2.00 | 2.00 |
| Water | q.s. to 100% | q.s. to 100% | q.s. to 100% |

The following procedures show how the above formulations are evaluated in terms of how well they clean greasy soil.

The first method used to evaluate grease cleaning of the compositions of this invention involves measuring interfacial tension (IFT) at the oil/water interface. IFT is a measure of the amount of energy needed to emulsify grease. The lower the number, the better the grease cleaning ability of the test product. IFT measurements are made on the University of Texas Model 500 Spinning Drop Interfacial Tensiometer. Solutions are prepared at the desired product concentration, water hardness and temperature. The usual conditions are 0.2% product concentration and 115°F (46.1°C) using animal fat. A narrow capillary tube is filled with the solution of the compositions and a drop of the melted animal fat is carefully added via a syringe. An IFT reading is taken immediately upon insertion of the sample into the tensiometer and again at five and ten minutes. Results for Formulations A, B, and C are shown in the table.

The second method involves gravimetric determination of the amount of solid animal fat removed from polypropylene cups under a soil situation. Between 3 and 8 grams of animal fat is solidified onto the bottom of polypropylene cups and 0.2-0.4% of the product is added. The % of fat removed after about 4 hours is a gauge of the grease cleaning efficacy of the compositions. Results for Formulation A, B, and C are shown below.

**Table I**

| | Performance Evaluation | |
|---|---|---|
| Composition | Interfacial Tension* (o/w, dynes/cm) | % Fat Removal** |
| A | 4.10 | 2.40 |
| B | 2.10 | 4.70 |
| C | 0.43 | 20.40 |

| | | |
|---|---|---|
| * Conditions: Animal fat, 0 wash water hardness, 0.2% product concentration | | |
| ** Conditions: Animal fat, 0 wash water hardness, 0.4% product concentration | | |

Results: Formulation C with calcium ions removes the most fat (20.40% versus 4.70% for the magnesium-containing formulation) and has the lowest Interfacial Tension, indicating better grease removal capacity. Formulation A with no calcium or magnesium ions removes the least amount of fat and has the highest Interfacial Tension. Formulation B with magnesium has values in between the results for Formulations A and C.

Conclusion: Both methods, IFT measurement and polypropylene cup grease removal, show that the calcium-containing formula, Formulation C, is much more effective at cleaning greasy soil in soft water (defined as wash water of 0-3 grams per gallon hardness) than the corresponding magnesium-containing formula, Formulation B, under the same conditions.

### EXAMPLE II

The following clear, stable, liquid compositions are formulated. The compositions are prepared in the same manner as the compositions of Example I.

| | % By Weight | | | | |
|---|---|---|---|---|---|
| Component | A | B | C | D | E |
| Ammonium C_{12/13} alkyl ethoxy (2.8 ave.) carboxylate | 10 | - | - | - | - |
| C₁₂ alkyl N-methyl glucamide | 6 | 9 | 12.7 | 16 | 12.8 |
| Ammonium C_{12/13} alkyl ethoxy (1.0 ave.) sulfate | 10 | 9 | 10 | - | 10 |
| Ammonium C_{12/13} alkyl ethoxy (3.0 ave.) sulfate | 4 | 14 | 2.7 | - | 2.7 |
| Ammonium C_{12/13} alkyl ethoxy (6.5 ave.) sulfate | - | 3 | - | 14 | - |
| C_{12/14} alkyl amine oxide | 1 | 1 | 1 | 1 | - |
| C_{12/14} alkylamido hydroxy sulfobetaine | 1 | 1 | 1 | - | - |
| C_{12/14} alkylamido propyl betaine | - | - | - | 3 | 2* |
| C₉₋₁₁ alkyl ethoxy (8-10 ave.) alcohol | - | - | 7 | - | 7 |
| Ethanol | 5 | 5 | 5 | 5 | 5 |
| Ammonium xylene sulfonate | 2 | 2 | 2 | 2 | 2** |
| Calcium chloride dihydrate | 3.7 | 3.7 | 1.85 | 3.70 | 1.85 |
| Others (water, perfume, dye) | Bal. | Bal. | Bal. | Bal. | Bal. |

| | | | | | |
|---|---|---|---|---|---|
| * Alkyl dimethyl betaine | | | | | |
| ** Sodium cumene sulfonate | | | | | |

### EXAMPLE III

The following clear, stable, liquid compositions are formulated using LSDA, magnesium ions, and/or with calcium ions. The compositions are formulated using the same manner as the compositions of Example I.

| | % By Weight | | | |
|---|---|---|---|---|
| Component | A | B | C | D |
| C₁₀₋₁₂ alkyl N-methyl glucamide | 9.00 | 11.00 | 11.00 | 16.00 |
| Ammonium C_{12/13} alkyl ethoxy (3.0 ave.) sulfates | 14.00 | 8.00 | 11.00 | --- |
| Ammonium C_{12/13} alkyl ethoxy (1.0 ave.) sulfates | 9.00 | 12.00 | --- | 14.00 |
| Ammonium C_{12/13} alkyl ethoxy (6.5 ave.) sulfates | 3.00 | 2.00 | --- | --- |
| C_{12/14} alkyl amine oxide | 1.00 | 1.00 | 2.50 | 1.00 |
| C_{12/14} alkyldimethyl betaine | --- | --- | --- | 3.00 |
| C_{12/14} alkylamido hydroxy sulfobetaine | 1.00 | 1.00 | 1.00 | --- |
| C₉₋₁₁ alkyl ethoxy (10 ave.) alcohol | --- | --- | 1.50 | --- |
| Calcium chloride dihydrate | 1.47 | 1.10 | 1.10 | 0.74 |
| Magnesium chloride hexahydrate | 5.53 | 5.95 | 5.95 | 5.28 |
| Ethanol | 5.00 | 5.00 | 5.00 | 5.00 |
| Ammonium xylene sulfonate | 2.00 | 2.00 | 2.00 | 2.00 |
| Others (perfume, dye, water, etc.) | Bal. | Bal. | Bal. | Bal. |

### EXAMPLE IV

The following heavy duty liquid detergent compositions are prepared.

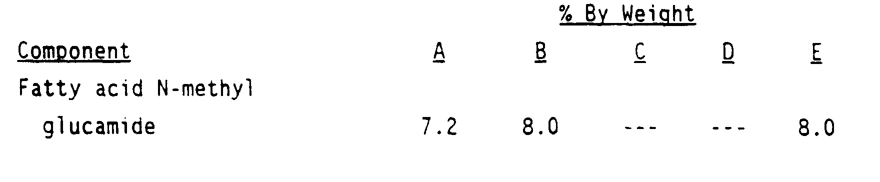

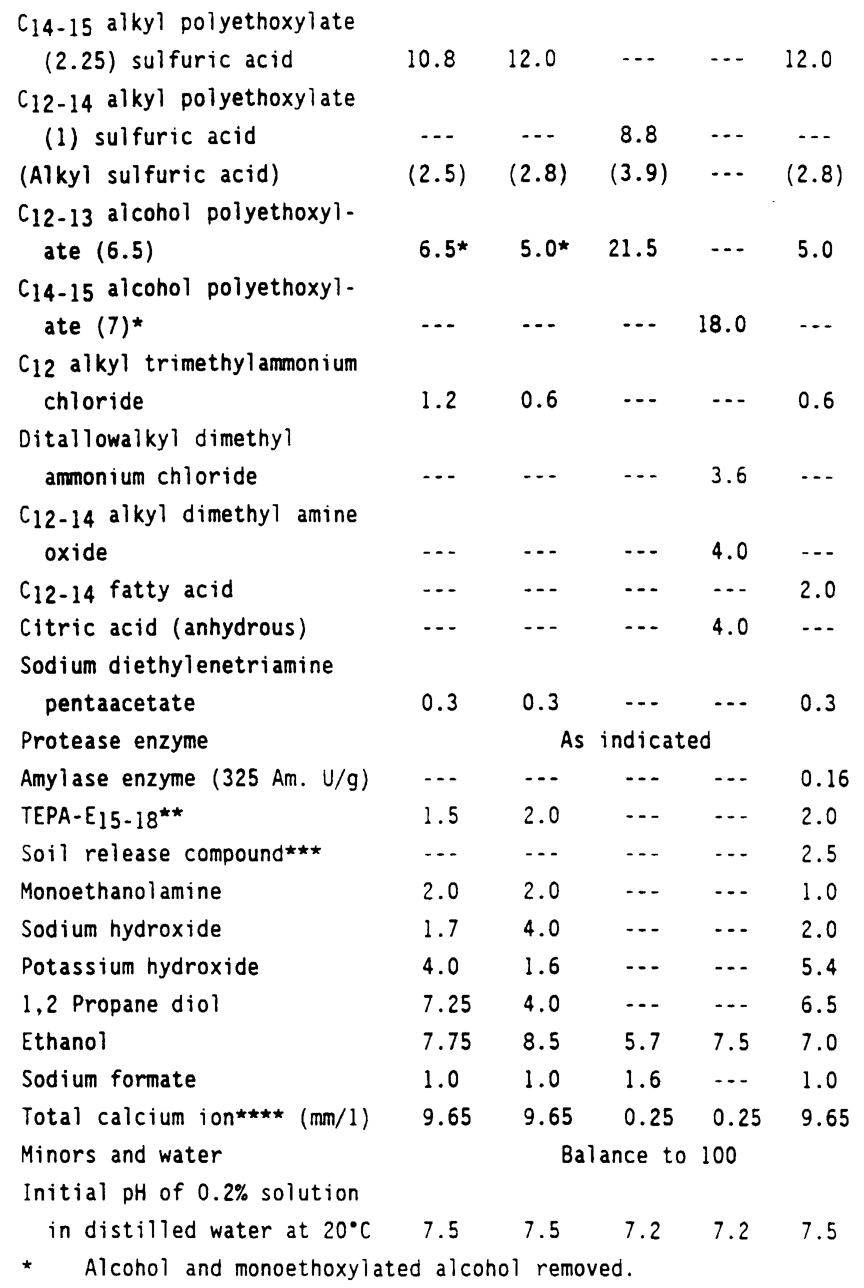

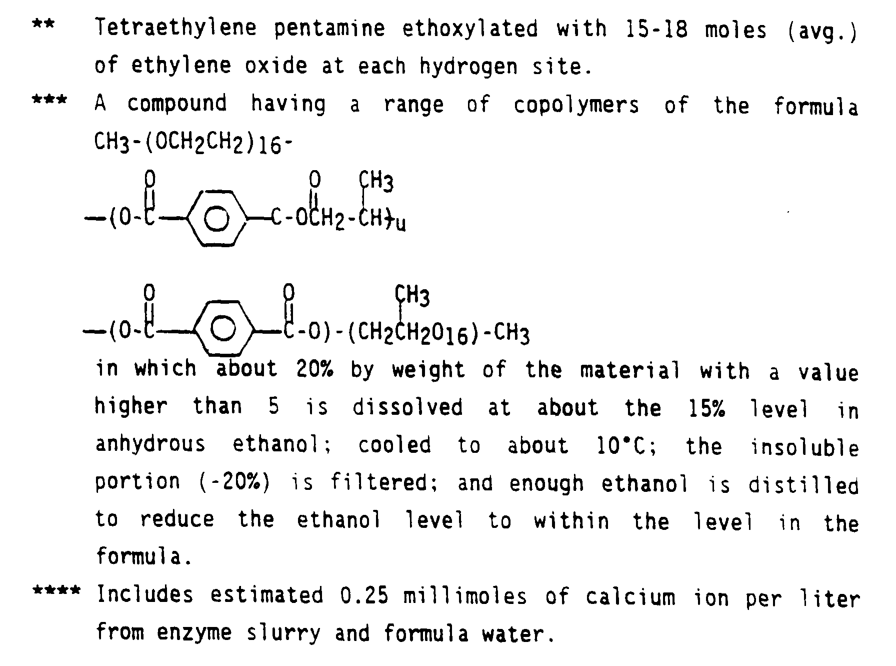

### EXAMPLE V

A light duty liquid dishwashing composition of the present invention is as follows.

| | % by Weight | | | | |
|---|---|---|---|---|---|
| Ingredients | A | B | C | D | E |
| C₁₂₋₁₄ alkyl ethoxy sulfate (1 EO) | 16 | 9 | 12 | -- | 16 |
| C₁₂₋₁₄ alkyl ethoxy sulfate (3 EO) | -- | 14 | -- | 11 | -- |
| C₁₀ alkyl ethoxylate (8EO) | 7 | 3 | 7 | 1 | 8 |
| C₁₂₋₁₄ N-methyl glucamide | 8 | 9 | 12 | 6 | 8 |
| Coconut diethanolamide | -- | -- | -- | 5 | -- |
| Dimethyl dodecyl amine oxide | -- | 1 | -- | 2 | -- |
| Cocoamidopropyl hydroxysulfobetaine | -- | 1 | 3 | -- | -- |
| Cocoamidopropyl betaine | 2 | -- | -- | -- | 2 |
| Magnesium ions | -- | -- | 1 | 1 | 0.7 |
| Calcium ions | 0.5 | 1 | -- | -- | 0.3 |
| Sodium toluene sulfonate | 3 | 3 | 3 | 3 | 2* |
| Ethanol | 4 | 4 | 4 | 4 | 4.5 |
| Water | ------ Balance ------ | | | | |

| | | | | | |
|---|---|---|---|---|---|
| * Sodium cumene sulfonate | | | | | |

### EXAMPLE VI

A granular laundry detergent composition of the present invention is as follows:

| Component | Active Weight % |
|---|---|
| Sodium C₁₄₋₁₅ alkyl ethoxy (2.5 ave.) sulfate | 12.80 |
| C₁₆₋₁₈ N-methyl glucamide | 12.80 |
| Sodium tripolyphosphate | 2.09 |
| Tetrasodium pyrophosphate | 17.44 |
| Sodium silicate | 7.04 |
| Polyethylene glycol | 0.25 |
| Sodium polyacrylate | 0.88 |
| Sodium perborate monohydrate | 4.32 |
| Sodium carbonate | 20.72 |
| Calcium sulfate dihydrate | 4.80 |
| Others (moisture, brightener, sodium sulfate) | Balance |

### EXAMPLE VII

A shampoo composition of the present invention is as follows:

| Component | |
|---|---|
| Ammonium C₁₂₋₁₄ alkyl sulfate | 12.00 |
| Sodium C₁₂₋₁₄ alkyl sulfate | 12.00 |
| C₁₂₋₁₄ N-methyl glucamide | 12.00 |
| C₁₂₋₁₄ alkyl amine oxide | 2.00 |
| C₁₂₋₁₄ alkyl diethanolamide | 1.00 |
| Calcium chloride dihydrate | 0.74 |
| Magnesium chloride hexahydrate | 2.50 |
| Panthanol* | 0.10 |
| Formaldehyde | 0.20 |
| C₁₂₋₁₈ hydroxysulfobetaine | 3.00 |
| Others (water, dye, perfume) | Balance |

| | |
|---|---|
| * 2.4-dihydroxy-N-(3-hydroxypropyl)-3.3 dimethylorityramide | |

### EXAMPLE VIII

A granular automatic dishwashing detergent of the present invention is as follows:

| Component | Wt. % |
|---|---|
| Sodium citrate | 15.0 |
| Sodium carbonate | 15.0 |
| Nonionic suds suppressor (1) | 4.0 |
| Sodium polyacrylate | 4.0 |
| Sodium silicate solids (SiO₂:Na₂O, 2.0 ratio) | 6.6 |
| Chlorine bleach solids (2) | 1.9 |
| Sodium C_{12/13} alkyl ethoxy (3.0 ave.) sulfate | 4.0 |
| C₁₂₋₁₈ N-methyl glucamide | 4.0 |
| Calcium sulfate dihydrate | 2.5 |
| Sodium sulfate, perfume, dye and water | Balance |

| | |
|---|---|
| (1) Blend of ethoxylated monohydroxy alcohol and polyoxyethylene/polyoxypropylene block polymer. Includes 3.2% monstearyl acid phosphate for suds suppression. | |
| (2) Sodium dichloroisocyanurate dihydrate. | |

## Claims

1. A detergent composition comprising, by weight of the composition:
(a) from 3% to 95% of anionic sulfate surfactant;
(b) from 3% to 40% of polyhydroxy fatty acid amide having the formula: 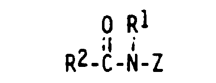 wherein R¹ is hydrogen, C₁-C₄ hydrocarbyl, 2-hydroxyethyl, 2-hydroxypropyl, or mixtures thereof; R² is C₅-C₃₁ hydrocarbyl; and Z is a polyhydroxy-hydrocarbyl having a linear hydrocarbyl chain with at least three hydroxyl groups directly connected to the chain, or an alkoxylated derivative thereof; and
(c) from 0.1% to 3% of calcium ions.

2. A composition according to Claim 1 comprising from 5% to 60% of said anionic sulfate surfactant which is selected from the group consisting of C₁₀-C₁₆ alkyl sulfate which has been ethoxylated with from 0.5 to 20 moles of ethylene oxide per molecule, C₉-C₁₇ acyl-N-(C₁-C₄ alkyl) glucamine sulfate, -N-(C₂-C₄ hydroxyalkyl) glucamine sulfate, and mixtures thereof.

3. A composition according to Claim 1 or 2 comprising from 5% to 30% of said polyhydroxy fatty acid amide, wherein R¹ is C₁-C₄ alkyl and R² is a straight-chain C₇-C₁₉ alkyl or alkenyl group or mixture thereof.

4. A composition according to any of the preceding claims comprising from 10% to 40% of C₁₀-C₁₆ alkyl sulfate which has been ethoxylated with from 0.5 to 20 moles of ethylene oxide per molecule; and comprising from 0.2% to 2% of calcium ions and having a pH in a 10% solution in water at 20°C of between 5.5 and 11.0; and where Z in said polyhydroxy fatty acid amide is derived from glucose or maltose or mixtures thereof.

5. A composition according to any of the preceding claims further comprising from 0.2% to 20% of lime soap dispersing agent, wherein said lime soap dispersing agent has a lime soap dispersant requirement of between 1 and 20; and comprising C₁₀-C₁₆ alkyl sulfate which has been ethoxylated with from 0.5 to 2.5 moles of ethylene oxide per molecule.

6. A liquid detergent composition according to any of the preceding claims wherein Z is selected from the group consisting of - CH₂- (CHOH)ₙ- CH₂OH,- CH(CH₂OH) - (CHOH)ₙ₋₁ - CH₂OH, -CH₂ - (CHOH)₂ (CHOR¹) (CHOH) - CH₂OH, where n is an integer from 3 to 5, inclusive, and R1 is H or a cyclic or aliphatic monosaccharide, and alkoxylated derivatives thereof.

7. A liquid composition according to any of the preceding claims comprising from 0.5% to 10% of C₈-C₂₂ sulfobetaine or hydroxysulfobetaine.

8. A liquid detergent composition according to any of the preceding claims comprising from 94% to 35% of a liquid carrier comprising a mixture of water and a C₁-C₄ monohydric alcohol; and having a pH in a 10% solution in water at 20°C of between 5.5 and 8.5; and wherein said lime soap dispersing agent has a lime soap dispersant requirement of between 2 and 10, and comprising from 1% to 6% of C₁₂-C₁₈ sulfobetaine or hydroxysulfobetaine.

9. A liquid detergent composition according to any of the preceding claims comprising from 0.3% to 1.5% of calcium ions added to the composition as calcium chloride; and further comprising from 0.05% to 1.5% by weight of magnesium ions; and wherein the polyhydroxy fatty acid amide is of the formula 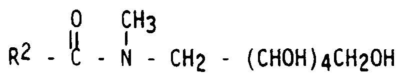 wherein R² is a straight-chain C₁₁-C₁₇ alkyl or alkenyl group.

10. A liquid detergent composition according to any of the preceding claims further comprising a calcium ion:magnesium ion of between 1:4 and 1:2, wherein said calcium and magnesium ions are added to said composition as chloride, hydroxide, oxide, acetate, or nitrate salts, or mixtures thereof.

11. A gel detergent composition according to any of the preceding claims further comprising from 0.1% to 10% by weight of polycarboxylate polymer and essentially no clay thickening agents.

12. A composition according to any of the preceding claims comprising from 5% to 65% of a surfactant mixture comprising said anionic sulfate surfactant and said polyhydroxy fatty acid amide.

13. A composition according to any of the preceding claims comprising from 1% to 15% by weight of additional nonionic surfactant selected from the group consisting of polyethylene, polypropylene and polybutylene oxide condensates of alkyl phenols; the alkyl ethoxylate condensation products of aliphatic alcohols with ethylene oxide; the condensation products of ethylene oxide with a hydrophobic base formed by the condensation of propylene oxide with propylene glycol; the condensation product of ethylene oxide with the product resulting from the reaction of propylene oxide and ethylenediamine; alkylpolysaccharides; fatty acid amides; and mixtures thereof.

14. A liquid detergent composition according to any of the preceding claims which is substantially free of chelating agent having a log stability constant above 8.0, and of a suds-suppressing amount of C₁₄ and higher fatty acid.

15. A composition according to any of Claims 1-5 wherein said composition additionally comprises from 2% to 5% by weight of a hydrotrope selected from the group consisting of sodium xylene sulfonate, ammonium xylene sulfonate, potassium xylene sulfonate, sodium toluene sulfonate, potassium toluene sulfonate, ammonium toluene sulfonate, sodium cumene sulfonate, potassium cumene sulfonate, ammonium cumene sulfonate, and mixtures thereof.

16. A granular detergent composition according to any of Claims 1-5 further comprising from 5% to 50% by weight of detergency builder.

17. A method for cleaning soiled dishes wherein said dishes are contacted with an effective amount of a detergent composition comprising, by weight of the composition:
(a) from 3% to 95% of anionic sulfate surfactant; and
(b) from 3% to 40% of polyhydroxy fatty acid amide having the formula 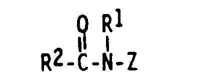 wherein R¹ is hydrogen, C₁-C₄ hydrocarbyl, 2-hydroxyethyl, 2-hydroxypropyl, or mixtures thereof; R² is C₅-C₃₁ hydrocarbyl, and Z is a polyhydroxy-hydrocarbyl having a linear hydrocarbyl chain with at least three hydroxyl groups directly connected to the chain, or an alkoxylated derivative thereof; and
(c) from 0.1% to 3% of calcium ions.

## Patentansprüche

1. Reinigungsmittelzusammensetzung, umfassend auf das Gewicht der Zusammensetzung bezogen:
(a) 3% bis 95% anionisches Sulfattensid;
(b) 3% bis 40% Polyhydroxyfettsäureamid der Formel: 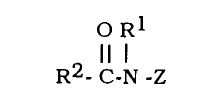 worin R¹ Wasserstoff, C₁-C₄-Hydrocarbyl, 2-Hydroxyethyl, 2-Hydroxypropyl oder Mischungen hiervon ist; R² C₅-C₃₁-Hydrocarbyl ist; und Z ein Polyhydroxyhydrocarbyl mit einer linearen Hydrocarbylkette, bei der mindestens drei Hydroxylgruppen direkt an die Kette gebunden sind, oder ein alkoxyliertes Derivat hiervon ist; und
(c) 0,1% bis 3% Calciumionen.

2. Zusammensetzung nach Anspruch 1, umfassend 5% bis 60% des anionischen Sulfattensids, welches aus der Gruppe gewählt ist, bestehend aus C₁₀-C₁₆-Alkylsulfat, das mit 0,5 bis 20 Molen Ethylenoxid pro Molekül ethoxyliert worden ist, C₉-C₁₇-Acyl-N-(C₁-C₄-alkyl)glucaminsulfat, -N-(C₂-C₄-Hydroxyalkyl)glucaminsulfat und Mischungen hiervon.

3. Zusammensetzung nach Anspruch 1 oder 2, umfassend 5% bis 30% des Polyhydroxyfettsäureamids, worin R¹ die Bedeutung C₁-C₄-Alkyl hat und R² eine geradkettige C₇-C₁₉-Alkyl- oder -alkenylgruppe oder eine Mischung hiervon ist.

4. Zusammensetzung nach mindestens einem der vorangehenden Ansprüche, umfassend 10% bis 40% C₁₀-C₁₆-Alkylsulfat, das mit 0,5 bis 20 Molen Ethylenoxid pro Molekül ethoxyliert worden ist; und umfassend 0,2% bis 2% Calciumionen, und welche einen pH in einer 10%igen Lösung in Wasser bei 20°C zwischen 5,5 und 11,0 aufweist; und worin Z in dem Polyhydroxyfettsäureamid von Glucose oder Maltose oder Mischungen hiervon abgleitet ist.

5. Zusammensetzung nach mindestens einem der vorangehenden Ansprüche, umfassend weiterhin 0,2% bis 20% Kalkseife-Dispergiermittel, wobei das Kalkseife-Dispergiermittel ein Kalkseife-Dispergiermittel-Erfodernis zwischen 1 und 20 aufweist; und umfassend C₁₀-C₁₆-Alkylsulfat, das mit 0,5 bis 2,5 Molen Ethylenoxid pro Molekül ethoxyliert worden ist.

6. Flüssige Reinigungsmittelzusammensetzung nach mindestens einem der vorangehenden Ansprüche, wobei Z aus der Gruppe gewählt ist, bestehend aus - CH₂- (CHOH)ₙ - CH₂OH,- CH (CH₂OH)-(CHOH)ₙ₋₁ - CH₂OH,- CH₂-(CHOH)₂ (CHOR¹) (CHOH) - CH₂OH, worin n eine ganze Zahl von 3 bis 5 einschließlich ist, und R¹ H oder ein cyclisches oder aliphatisches Monosaccharid, und alkoxylierte Derivate hiervon ist.

7. Flüssige Zusammensetzung nach mindestens einem der vorangehenden Ansprüche, umfassend 0,5 bis 10% C₈-C₂₂-Sulfobetain oder -Hydroxysulfobetain.

8. Flüssige Reinigungsmittelzusammensetzung nach mindestens einem der vorangehenden Ansprüche, umfassend 94% bis 35% eines flüssigen Trägers, umfassend eine Mischung aus Wasser und einem einwertigen C₁-C₄-Alkohol; und welche einen pH in einer 10%igen Lösung in Wasser bei 20°C zwischen 5,5 und 8,5 aufweist; und wobei das Kalkseife-Dispergiermittel ein Kalkseife-Dispergiermittel-Erfordernis zwischen 2 und 10 aufweist, und umfassend 1% bis 6% C₁₂-C₁₈-Sulfobetain oder -Hydroxysulfobetain.

9. Flüssige Reinigungsmittelzusammensetzung nach mindestens einem der vorangehenden Ansprüche, umfassend 0,3% bis 1,5% Calciumionen, welche der Zusammensetzung als Calciumchlorid zugesetzt sind; und umfassend weiterhin 0,05 bis 1,5 Gew.-% Magnesiumionen; und wobei das Polyhydroxyfettsäureamid der Formel 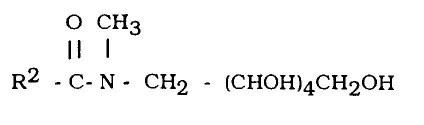 entspricht, worin R² eine geradkettige C₁₁-C₁₇-Alkyl oder -Alkenylgruppe ist.

10. Flüssige Reinigungsmittelzusammensetzung nach mindestens einem der vorangehenden Ansprüche, wobei weiterhin das Verhältnis Calciumionen:Magnesiumionen zwischen 1:4 und 1:2 liegt, wobei die Calcium- und Magnesiumionen der Zusammensetzung als Chlorid-, Hydroxid-, Oxid-, Acetat- oder Nitratsalze oder Mischungen hiervon zugesetzt sind.

11. Gelförmige Reinigungsmittelzusammensetzung nach mindestens einem der vorangehenden Ansprüche, umfassend weiterhin 0,1 bis 10 Gew.-% Polycarboxylatpolymer und im wesentlichen keine Tonverdickungsmittel.

12. Zusammensetzung nach mindestens einem der vorangehenden Ansprüche, umfassend 5% bis 65% einer Tensidmischung, umfassend das anionische Sulfattensid und das Polyhydroxyfettsäureamid.

13. Zusammensetzung nach mindestens einem der vorangehenden Ansprüche, umfassend 1 bis 15 Gew. -% zusätzliches nichtionisches Tensid, gewählt aus der Gruppe, bestehend aus Polyethylen-, Polypropylen- und Polybutylenoxid-Kondensaten von Alkylphenolen; den Alkylethoxylat-Kondensationsprodukten von aliphatischen Alkoholen mit Ethylenoxid; den Kondensationsprodukten von Ethylenoxid mit einer hydrophoben Base, gebildet durch die Kondensation von Propylenoxid mit Propylenglykol; den Kondensationsprodukten von Ethylenoxid mit dem aus der Umsetzung von Propylenoxid und Ethylendiamin resultierenden Produkt; Alkylpolysacchariden; Fettsäureamiden; und Mischungen hiervon.

14. Flüssige Reinigungsmittelzusammensetzung nach mindestens einem der vorangehenden Ansprüche, welche im wesentlichen frei ist an Komplexbildner mit einer log-Stabilitätskonstante oberhalb 8,0, sowie an einer schaumunterdrückenden Menge an C₁₄- und höherer Fettsäure.

15. Zusammensetzung nach mindestens einem der Ansprüche 1-5, wobei die Zusammensetzung zusätzlich 2 bis 5 Gew.-% eines Hydrotrops umfaßt, gewählt aus der Gruppe, bestehend aus Natriumxylolsulfonat, Ammoniumxylolsulfonat, Kaliumxylolsulfonat, Natriumtoluolsulfonat, Kaliumtoluolsulfonat, Ammoniumtoluolsulfonat, Natriumcumolsulfonat, Kaliumcumolsulfonat, Ammoniumcumolsulfonat und Mischungen hiervon.

16. Granuläre Reinigungsmittelzusammensetzung nach mindestens einem der Ansprüche 1-5, umfassend weiterhin 5 bis 50 Gew. -% eines Waschmittelbuilders.

17. Verfahren zum Reinigen von verschmutztem Geschirr, wobei das Geschirr mit einer wirksamen Menge einer Reinigungsmittelzusammensetzung kontaktiert wird, umfassend, bezogen auf das Gewicht der Zusammensetzung:
(a) 3% bis 95% anionisches Sulfattensid; und
(b) 3% bis 40% Polyhydroxyfettsäureamid der Formel: 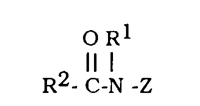 worin R¹ Wasserstoff, C₁-C₄-Hydrocarbyl, 2-Hydroxyethyl, 2-Hydroxypropyl oder Mischungen hiervon ist; R² C₅-C₃₁-Hydrocarbyl ist; und Z ein Polyhydroxyhydrocarbyl mit einer linearen Hydrocarbylkette, bei der mindestens drei Hydroxylgruppen direkt an die Kette gebunden sind, oder ein alkoxyliertes Derivat hiervon ist; und
(c) 0,1% bis 3% Calciumionen.

## Revendications

1. Composition détergente comprenant, en poids par rapport à la composition :
(a) de 3 % à 95 % d'un tensioactif sulfate anionique ;
(b) de 3 % à 40 % d'un amide d'acide gras polyhydroxylé ayant pour formule 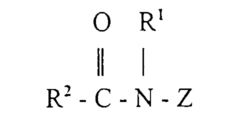 dans laquelle R¹ est un atome d'hydrogène ou un groupe hydrocarbyle en C₁-C₄, 2-hydroxyéthyle, 2-hydroxypropyle ou des mélanges de ceux-ci ; R² est un groupe hydrocarbyle en C₅-C₃₁ ; et Z est un groupe hydrocarbyle polyhydroxylé ayant une chaîne hydrocarbyle linéaire comportant au moins trois groupes hydroxyle directement liés à la chaîne, ou un de ses dérivés alcoxylés ; et
(c) de 0,1 % à 3 % d'ions calcium.

2. Composition selon la revendication 1, comprenant de 5 % à 60 % dudit tensioactif sulfate anionique, qui est choisi dans l'ensemble comprenant les alkylsulfates en C₁₀-C₁₆ qui ont été éthoxylés avec 0,5 à 20 moles d'oxyde d'éthylène par molécule, les (acyl en C₉-C₁₇)-N-(alkyl en C₁-C₄)-glucaminesulfates, les -N-(hydroxyalkyl en C₂-C₄)-glucaminesulfates, et leurs mélanges.

3. Composition selon la revendication 1 ou 2, qui comprend de 5 % à 30 % dudit amide d'acide gras polyhydroxylé, où R' est un groupe alkyle en C₁-C₄ et R² est un groupe alkyle ou alcényle en C₇-C₁₉ à chaîne droite, ou un de leurs mélanges.

4. Composition selon l'une quelconque des revendications précédentes, comprenant de 10 % à 40 % d'un alkylsulfate en C₁₀-C₁₆ qui a été éthoxylé avec 0,5 à 20 moles d'oxyde d'éthylène par molécule ; et comprenant de 0,2 % à 2 % d'ions calcium et ayant un pH en solution aqueuse à 10 % à 20°C compris entre 5,5 et 11,0 ; et où Z, dans ledit amide d'acide gras polyhydroxylé, dérive du glucose, ou du maltose, ou de leurs mélanges.

5. Composition selon l'une quelconque des revendications précédentes, comprenant en outre de 0,2 % à 20 % d'un agent dispersant de savon de chaux, où ledit agent dispersant de savon de chaux a un besoin en dispersant de savon de chaux compris entre 1 et 20 ; et comprenant un alkylsulfate en C₁₀-C₁₆ qui a été éthoxylé avec 0,5 à 2,5 moles d'oxyde d'éthylène par molécule.

6. Composition détergente liquide selon l'une quelconque des revendications précédentes, dans laquelle Z est choisi dans l'ensemble comprenant les groupes -CH₂-(CHOH)ₙ-CH₂OH, -CH(CH₂OH)-(CHOH)ₙ₋₁-CH₂OH, -CH₂-(CHOH)₂(CHOR¹)(CHOH)-CH₂OH, où n est un entier de 3 à 5, limites comprises, et R¹ est H ou un monosaccharide cyclique ou aliphatique, et leurs dérivés alcoxylés.

7. Composition liquide selon l'une quelconque des revendications précédentes comprenant de 0,5 % à 10 % d'une sulfobétaïne ou d'une hydroxysulfobétaïne en C₈-C₂₂.

8. Composition détergente liquide selon l'une quelconque des revendications précédentes, comprenant de 94 % à 35 % d'un support liquide comprenant un mélange d'eau et d'un monoalcool en C₁-C₄ ; et ayant un pH en solution aqueuse à 10 % à 20°C compris entre 5,5 et 8,5 ; et où l'agent dispersant de savon de chaux a un besoin en dispersant de savon de chaux compris entre 2 et 10, et comprenant de 1 % à 6 % d'une sulfobétaïne ou d'une hydroxysulfobétaïne en C₁₂-C₁₈.

9. Composition détergente liquide selon l'une quelconque des revendications précédentes, comprenant de 0,3 % à 1,5 % d'ions calcium ajoutés à la composition sous forme de chlorure de calcium ; et comprenant en outre de 0,05 % à 1,5 % en poids d'ions magnésium ; et dans laquelle l'amide d'acide gras polyhydroxylé a pour formule : 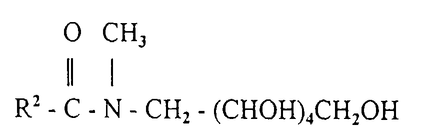 dans laquelle R² est un groupe alkyle ou alcényle en C₁₁-C₁₇ à chaîne droite.

10. Composition détergente liquide selon l'une quelconque des revendications précédentes, qui comprend en outre un rapport ions calcium:ions magnésium compris entre 1:4 et 1:2, où lesdits ions calcium et magnésium sont ajoutés à ladite composition sous forme de sels chlorures, hydroxydes, oxydes, acétates ou nitrates, ou leurs mélanges.

11. Composition détergente en gel selon l'une quelconque des revendications précédentes, qui comprend en outre de 0,1 % à 10 % en poids d'un polymère de polycarboxylate et essentiellement aucun agent épaississant de type argile.

12. Composition selon l'une quelconque des revendications précédentes, qui comprend de 5% à 65 % d'un mélange de tensioactifs comprenant ledit tensioactif sulfate anionique et ledit amide d'acide gras polyhydroxylé.

13. Composition selon l'une quelconque des revendications précédentes, qui comprend de 1 % à 15 % en poids d'un tensioactif non ionique additionnel choisi dans l'ensemble comprenant les produits de condensation polyoxyéthylénés, polyoxypropylénés et polyoxybutylénés des alkylphénols ; les produits de condensation alcoxylés d'alcools aliphatiques avec l'oxyde d'éthylène ; les produits de condensation de l'oxyde d'éthylène avec une base hydrophobe formée par condensation d'oxyde de propylène avec du propylèneglycol ; le produit de condensation de l'oxyde d'éthylène avec le produit résultant de la réaction de l'oxyde de propylène et de l'éthylènediamine ; les alkylpolysaccharides ; les amides d'acides gras ; et leurs mélanges.

14. Composition détergente liquide selon l'une quelconque des revendications précédentes, qui est pratiquement exempte d'agent chélatant ayant une constante de stabilité logarithmique supérieure à 8,0, et d'une quantité à effet antimoussant d'un acide gras en C₁₄ et plus.

15. Composition selon l'une quelconque des revendications 1 à 5, dans laquelle ladite composition comprend en outre de 2 % à 5 % en poids d'un hydrotrope choisi dans l'ensemble comprenant le xylènesulfonate de sodium, le xylènesulfonate d'ammonium, le xylènesulfonate de potassium, le toluènesulfonate de sodium, le toluènesulfonate de potassium, le toluènesulfonate d'ammonium, le cumènesulfonate de sodium, le cumènesulfonate de potassium, le cumènesulfonate d'ammonium et leurs mélanges.

16. Composition détergente granulaire selon l'une quelconque des revendications 1 à 5, qui comprend en outre de 5 % à 50 % en poids d'un adjuvant de détergence.

17. Procédé pour nettoyer des articles de vaisselle salis, où lesdits articles de vaisselle sont mis en contact avec une quantité efficace d'une composition détergente comprenant, en poids par rapport à la composition :
(a) de 3 % à 95 % d'un tensioactif sulfate anionique ;
(b) de 3 % à 40 % d'un amide d'acide gras polyhydroxylé ayant pour formule 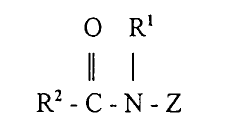 dans laquelle R¹ est un atome d'hydrogène ou un groupe hydrocarbyle en C₁-C₄, 2-hydroxyéthyle, 2-hydroxypropyle ou des mélanges de ceux-ci ; R² est un groupe hydrocarbyle en C₅-C₃₁ ; et Z est un groupe hydrocarbyle polyhydroxylé ayant une chaîne hydrocarbyle linéaire comportant au moins trois groupes hydroxyle directement liés à la chaîne, ou un de ses dérivés alcoxylés ; et
(c) de 0,1 % à 3 % d'ions calcium.
